(19) Europäisches Patentamt
European Patent Office
Office européen des brevets

(11) Publication number: 0 207 913 B1

(12) **EUROPEAN PATENT SPECIFICATION**

(45) Date of publication of patent specification: **31.10.90**

(51) Int. Cl.$^5$: **C 07 D 207/09**, C 07 C 65/21, A 61 K 31/40

(21) Application number: **86850209.7**

(22) Date of filing: **12.06.86**

(54) **Catechol carboxamides, process for their preparation, intermediates and a pharmaceutical preparation.**

(30) Priority: **19.06.85 SE 8503054**

(43) Date of publication of application:
**07.01.87 Bulletin 87/02**

(45) Publication of the grant of the patent:
**31.10.90 Bulletin 90/44**

(84) Designated Contracting States:
**AT BE CH DE FR GB IT LI LU NL SE**

(56) References cited:
**EP-A-0 004 831**
**EP-A-0 060 235**
**EP-A-0 105 599**
**DE-A-2 508 045**
**DE-A-3 334 594**
**FR-M- 5 916**
**US-A-3 342 826**

(73) Proprietor: **Astra Läkemedel Aktiebolag**
**Strängnäsvägen 44**
**S-151 85 Södertälje (SE)**

(72) Inventor: **Hall, Hakan Olof**
**Lingonvägen 20**
**S-155 00 Nykvarn (SE)**
Inventor: **Högberg, Thomas**
**Batmansvägen 28**
**S-151 39 Södertälje (SE)**
Inventor: **de Paulis, Tomas**
**Vanderbilt University, P O Box 1619, Sta. B**
**Nashville Tennessee 37235 (US)**
Inventor: **Ström, Hans Eric Peter**
**Parkvägen 3C II**
**S-153 00 Järna (SE)**
Inventor: **Ögren, Sven Ove**
**Hökmossvägen 14B**
**S-155 00 Nykvarn (SE)**

(74) Representative: **Danielsson, Sten Ove et al**
**AB ASTRA Patent and Trademark Department**
**S-151 85 Södertälje (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Field of the Invention

The present invention relates to novel, neuropharmacologically active carboxamide derivatives of catechol, processes for their preparation, pharmaceutical compositions containing the carboxamide derivatives and the methods of their pharmacological use.

The object of the invention is to provide a substituted benzamide neuroleptic useful for the blockade of dopamine receptors in the brain. Such substances will be useful in the treatment of emesis, anxiety states, psychosomatic diseases and psychotic states, such as schizophrenia and depression, alcoholic related diseases, confusional states and sleep disturbances in the elderly.

Prior art

From US patent 3342826 the compound of the formula

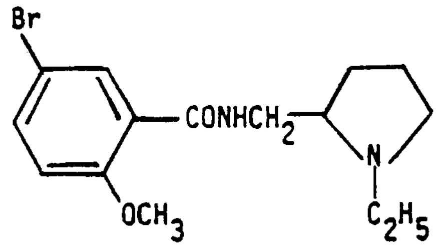

is known. This compound is claimed to be an inhibitor of the conditioned avoidance behaviour in the rat.

From US patent 4232037 the compound of the formula

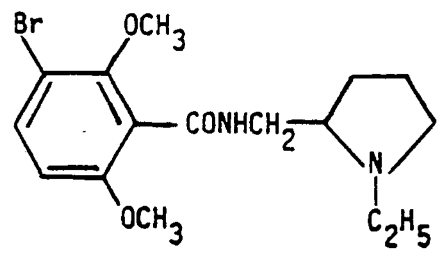

is known. This compound is claimed to be an antagonist of the apomorphine syndrome in the rat.

From Belg. patent 856 289 the compound of the formula

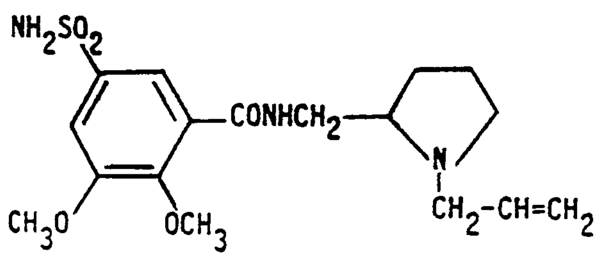

is known. This compound is claimed to have endocrinal activity in man.

From UK patent application 2088364 the compound of the formula

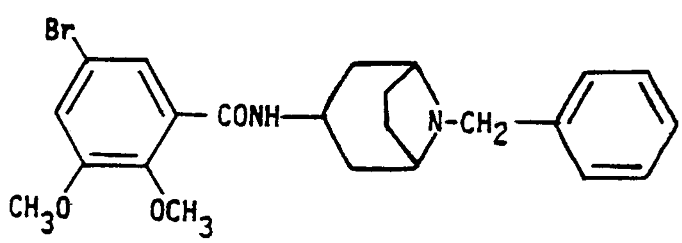

is known. This compound is claimed to be an inhibitor of the apomorphine syndrome in the mouse, and to possess anti-emetic effect in the dog.

From European patent application EP 60235 the compound of the formula

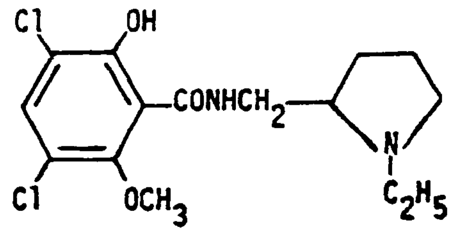

is known. This compound is claimed to be an inhibitor of the apomorphine syndrome in the rat.

From Norwegian patent 131 834 the compound of the formula

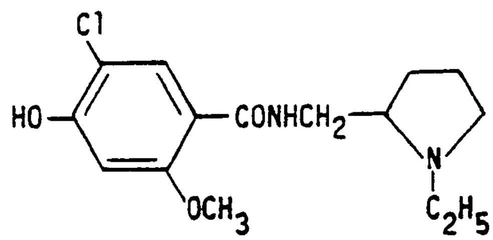

is known. This compound is claimed to have anti-emetic effect.

From French patent application 2 534 255 the compound of the formula

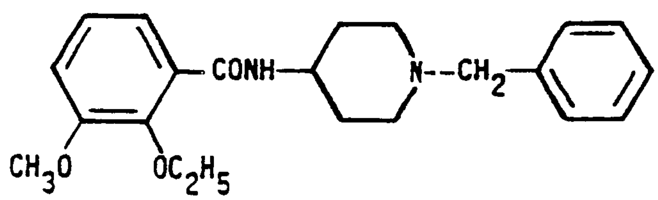

is known. This compound is claimed to antagonize the apomorphine syndrome.

From French patent 5 916 M the compound of the formula

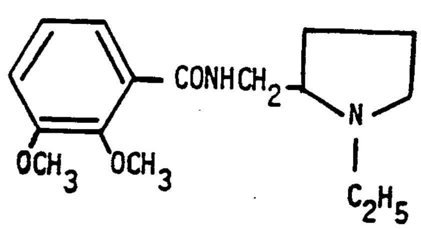

is known. This compound is claimed to have anti-emetic effect.

From DE 25 08045 the compounds of the formula

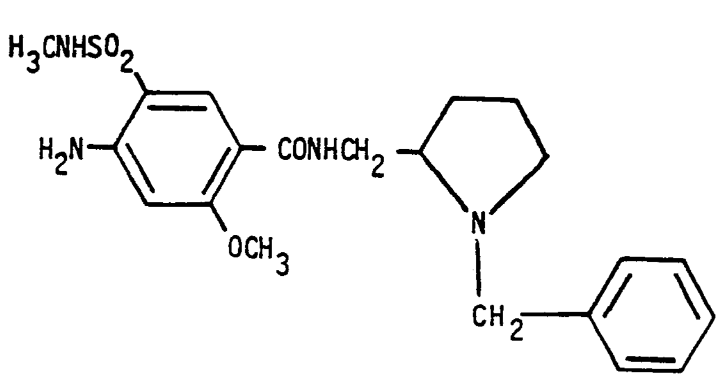

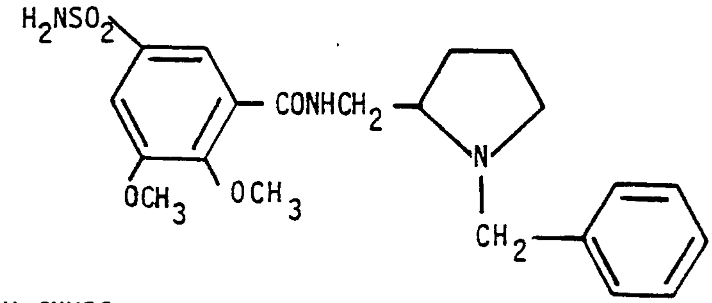

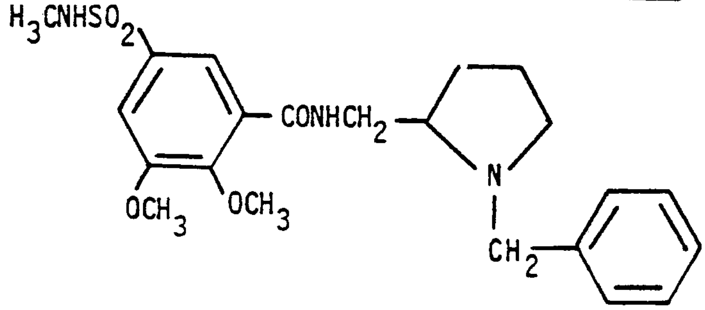

are known. These compounds are claimed to have anti-emetic effect, anti-ulcus effect and effect on the central nervous system.

Disclosure of the Invention

The present invention describes new compounds useful for treating mental disturbances such as psychotic states and schizophrenia. These agents are potent in their ability to block model psychotic states in the rat induced by the dopamine agonist apomorphine and/or in their activity to block the dopamine ligand spiperone. Furthermore these compounds possess a favourable profile with respect to drug induced side effects, such as extrapyramidal effects.

For instance the agents of this invention are favourable regarding their separation between the dose that inhibits the apomorphine-induced stereotypy, which might reflect undesirable side effects on motor centra in the brain, and the dose that inhibits the apomorphine induced hypermotility associated with blockade of dopamine receptors in the limbic areas of the brain.

The compounds of the invention are characterized by the general formula

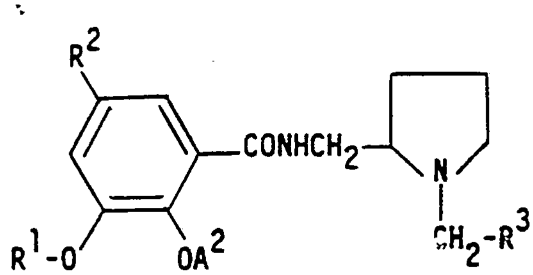

I

wherein

$R^1$ is a hydrogen atom, an alkyl(1—4C) group, an alkyl(1—20C)—CO group, an alkyl(1—20C)—O—CO group, an alkyl(1—20C)—NH—CO group or an

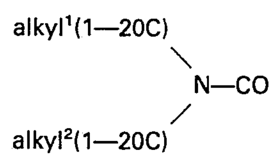

group;

$R^2$ is a halogen atom, an alkyl(1—4C) group or an alkyl(1—4C)-thio group;

$A^2$ is a methyl or ethyl group; and

$R^3$ is a hydrogen atom, an alkyl(1—4C) group, an alkenyl(2—3C) group, or a substituted phenyl group; or a physiologically acceptable salt or optical isomer thereof.

The invention thus provides compounds, and physiologically acceptable salts thereof, which are useful in therapeutic treatment of emesis, anxiety states, psychosomatic diseases such as gastric and duodenal ulcer, and psychotic states such as schizophrenia and depression, alcoholic related diseases, confusional states and sleep disturbances in the elderly.

Halogen atoms in formula I comprises chlorine, bromine, fluorine and iodine atoms.

Alkyl groups in formula I are straight or branched alkyl groups with 1 to 4 carbon atoms, i.e. methyl, ethyl, n-propyl, i-propyl, n-butyl, s-butyl, i-butyl and t-butyl.

Alkylthio groups in the formula I are groups —S-alkyl, wherein the alkyl moiety is defined as alkyl above.

Alkenyl groups in formula I are straight or branched hydrocarbon chains with 2 to 3 carbon atoms and a double bond, such as vinyl, allyl or isopropenyl.

Alkoxycarbonyl groups in formula I are alkyl-O—CO wherein the alkyl moiety is a straight or branched hydrocarbon chain with 1—20C, preferably 1—15 carbon atoms.

Monoalkylcarbamoyl groups in formula I are groups alkyl-NH—CO— wherein the alkyl moiety is a straight or branched hydrocarbon chain with 1—20C, preferably 1—15 carbon atoms.

Dialkylcarbamoyl groups in formula I are groups

```
alkyl¹
      \
       N—CO—
      /
alkyl²
```

wherein the $alkyl^1$ and $alkyl^2$ can be the same of different and each is straight or branched hydrocarbon chain with 1—20C, preferably 1—15 carbon atoms.

Acyl groups in formula I are alkyl-CO— where the alkyl moiety is a straight or branched hydrocarbon chain with 1 to 20 carbon atoms, preferably 1—15 carbon atoms.

Substituted phenyl in the formula I is a phenyl group substituted by one or more of fluoro, chloro, bromo, trifluoromethyl, ethyl, methoxy or ethoxy in the ortho, meta or para positions, or substituted by methylenedioxy.

Phenyl substituted with methylenedioxy in formula I is the group

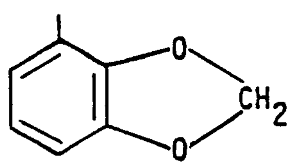 or 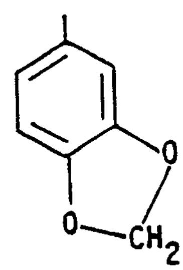

A preferred group of compounds of the invention is obtained when in formula I
$R^1$ is hydrogen, methyl, ethyl or acyl;
$R^2$ is halogen or alkyl;
$A^2$ is methyl or ethyl; and
$R^3$ has the definition given above.

A second preferred group of compounds of the invention is obtained when in formula I
$R^1$ is hydrogen, methyl or ethyl;
$R^2$ is chlorine, bromine, iodine, methyl, ethyl, n-propyl or n-butyl;
$A^2$ is methyl or ethyl; and
$R^3$ has the definition given above.

A third preferred group of compounds of the invention is obtained when in formula I
$R^1$ is methyl;
$R^2$ is chlorine, bromine, iodin, methyl, ethyl, n-propyl or n-butyl;
$A^2$ is methyl; and
$R^3$ is a hydrogen atom, an alkyl(1—4C) group, an alkenyl(2—3C) group or a phenyl group substituted in the para position with a fluorine atom.

A further preferred group of compounds of the invention is obtained when in formula I
$R^1$ is methyl;
$R^2$ is bromine;
$A^2$ is methyl;
$R^3$ is a hydrogen atom, an alkyl(1—4C) group, an alkenyl(2—3C) group or a phenyl group substituted in the para position with a fluorine atom.

Compounds particularly preferred are

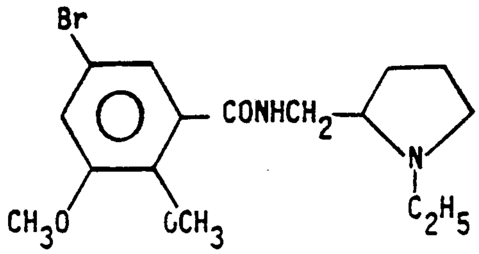 ; 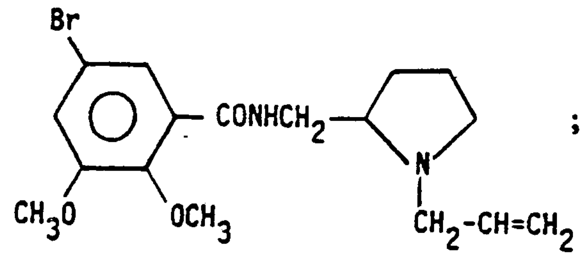 ;

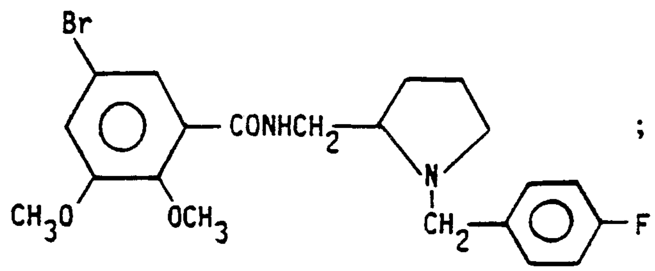 ; 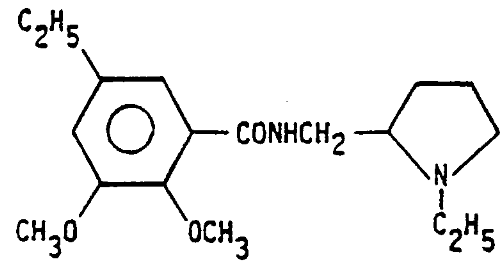 ;

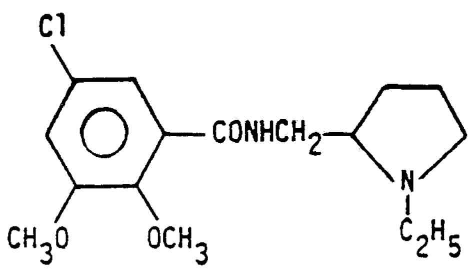 ; 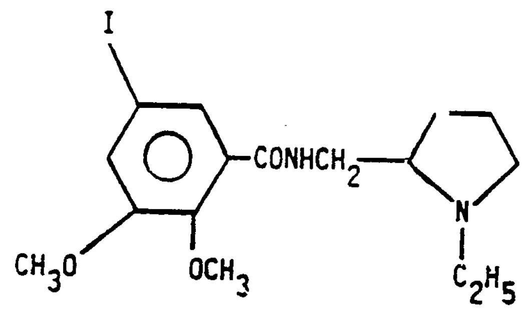 ;

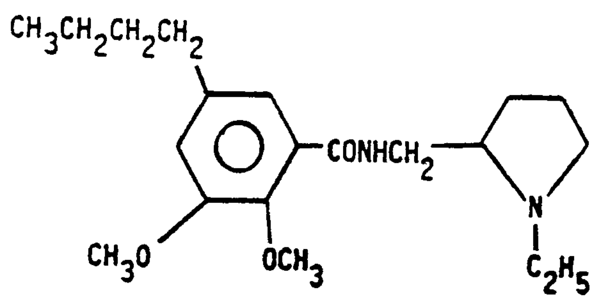

The new compounds of this invention may be used therapeutically as the racemic mixtures of (+)- and (−)-forms, which are obtained by synthesis. They may also be resolved into the corresponding enantiomers which, likewise, may be used in therapy. The (+)- and (−)-forms may also be obtained by the reaction of the corresponding enantiomeric 2-(amino-methyl)pyrrolidine with the benzoic acid moiety. Preferably $R^3$ = alkyl or alkenyl the configuration being S (sinister) $R^3$ = substituted phenyl the configuration being R (rectus).

Pharmaceutical preparations

In clinical practice the compounds of the present invention will normally be administered orally, rectally or by injection in the form of pharmaceutical preparations comprising the active ingredient either as a free base or as a pharmaceutically acceptable non-toxic, acid addition salt, e.g. the hydrobromide, hydrochloride, phosphate, sulphate, sulphonate, sulphamat, citrate, lactate, maleate, tartrate, acetate and the like in association with a pharmaceutically acceptable carrier. Accordingly, terms relating to the novel compounds of this invention whether generically or specifically are intended to include both the free amine base and the acid addition salts of the free base, unless the context in which such terms are used, e.g. in the specific examples would be inconsistent with the broad concept.

The carrier may be a solid, semisolid or liquid diluent or capsule. These pharmaceutical preparations constitute a further aspect of this invention. Usually the active substance will constitute between 0.1 and 99% by weight of the preparation, more specifically between 0.5 and 20% by weight for preparations

intended for injection between 2 and 50% by weight for preparations suitable for oral administration.

To produce pharmaceutical preparations containing a compound of the invention in the form of dosage units for oral application, the selected compound may be mixed with a solid pulverulent carrier, e.g. lactose, saccharose, sorbitol, mannitol, starches such as potato starch, corn starch or amylopectin, cellulose derivatives, or gelatine, and a lubricant such as magnesium stearate, calcium stearate, polyethylene glycol waxes, and the like, and then compressed to form tablets. If coated tablets are required, the cores, prepared as described above, may be coated with a concentrated sugar solution which may contain, e.g. gum arabic, gelatine, talcum titanium dioxide, and the like.

Alternatively, the tablet can be coated with a lacquer dissolved in a readily volatile organic solvent or mixture of organic solvents. Dyestuffs may be added to these coatings in order to readily distinguish between tablets containing different active substances or different amounts of the active compound.

For the preparation of soft gelatine capsules (pearl-shaped closed capsules) consisting of gelatine and for example, glycerol or similar closed capsules, the active substance may be admixed with a vegetable oil. Hard gelatine capsules may contain granulates of the active substance in combination with solid-pulverulent carriers such as lactose, saccharose, sorbitol, mannitol, starches (e.g. potato starch, corn starch or amylopectin), cellulose derivatives or gelatine.

Dosage units for rectal application can be prepared in the form of suppositories comprising the active substance in admixture with a neutral fatty base, or gelatine rectal capsules comprising the active substance in admixture with vegetable oil or paraffin oil.

Liquid preparations for oral application may be in the form of syrups or suspensions, for example solutions containing from about 0.2 to about 20% by weight of the active substance herein described, the balance being sugar and a mixture of ethanol, water, glycerol, and propyleneglycol. Optionally such liquid preparations may contain colouring agents, flavouring agents, saccharine and carboxymethylcellulose as a thickening agent.

Solutions for parenteral applications by injection can be prepared in an aqueous solution of a watersoluble pharmaceutically acceptable salt of the active substance preferably in a concentration of from about 0.5 to about 10% by weight. These solutions may also contain stabilizing agents and/or buffering agents and may conveniently be provided in various dosage unit ampoules.

Compounds of the formula I wherein $R^1$ is an acyl group, and alkoxycarbonyl group or a mono- or dialkylcarbamoyl group may advantageously be used in pharmaceutical preparations intended for intramuscular administration in order to obtain a sustained release effect, that is a depot effect.

Suitable daily doses for oral administration of the compounds of this invention are 1—50 mg, preferably 5—20 mg.

Methods of preparation

The compounds of the invention may be obtained by one of the following methods.

A. The compounds of the formula

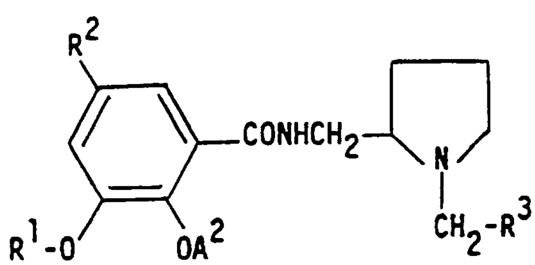

wherein

$R^1$ is a hydrogen atom, an alkyl(1—4C) group, an alkyl(1—20C)—CO group, an alkyl(1—20C)—O—CO group, and alkyl(1—20C)—NH—CO group or an

alkyl$^1$(1—20C)
\
N—CO
/
alkyl$^2$(1—20C)

group;

$R^2$ is a halogen atom, an alkyl(1—4C) group, or an alkyl(1—4C)thio group;

$A^2$ is a methyl or ethyl group; and

$R^3$ is a hydrogen atom, an alkyl(1—4C) group, an alkenyl(2—3C) group, or a substituted phenyl group, can be obtained by reaction of a compound of the formula

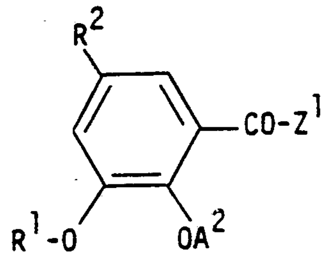

wherein $R^1$, $R^2$ and $A^2$ have the above given definitions and —CO—$Z^1$ is a reactive group capable of reacting with an amino group under formation of an amide moiety, with a compound of the formula

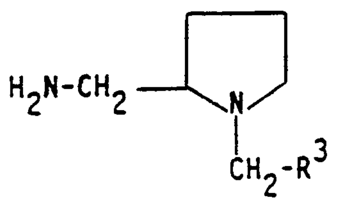

wherein $R^3$ has the above given definition, or a reactive derivative thereof.

The reaction is carried out in a suitable solvent, such as diethyl ether, THF, dichloromethane, chloroform or toluene between 0°C and the boiling point of the reaction mixture. The resulting amine can be isolated as a salt recovered e.g. by filtration. Alternatively, the amine obtained can be converted to the free base using conventional techniques, such as the addition of aqueous ammonia or a sodium hydroxide solution and extraction with an organic solvent.

$Z^1$ in the acylating group —CO—$Z^1$ may be a halogen group, such as chlorine or bromine, a mixed anhydride with inorganic acids or their esters (e.g. phenylphosphate), a thio group, an organic residue or a hydroxy group in combination with a coupling agent or reactive amine derivative.

The organic residue comprises groups which can form reactive acid derivatives. These can be aliphatic esters, e.g. methyl, ethyl, cyanomethyl or methoxymethyl esters, N-hydroxyimide esters or substituted or unsubstituted aromatic esters; acyl nitrile; acyl azide; symmetrical anhydrides; mixed anhydrides; or azolides, e.g. triazolide, tetrazolide or imidazolide.

According to the invention the following compounds can be used as reactive derivatives of the cyclic amine above:

Reaction products of the amine with phosphorus chloride, phosphorus oxychloride, dialkyl, diaryl or o-phenylenechlorophosphites or alkyl or aryldichlorophosphites, or an isothiocyanate or isocyanate of the amine. The mentioned reactive derivatives can be reacted with the acid *in situ* or after previous isolation.

It is also possible to react the free acid and the free amine in the presence of a condensating agent, e.g. silicon tetrachloride, diphosphoruspentoxide, a phosphine or hexamethylphosphorus triamide plus a carbon tetrahalide, diphenyl phosphite, N-ethoxycarbonyl-2-ethoxy-1,2-dihydroquinoline, titanium tetrachloride, or carbodiimides such as dicyclohexylcarbodiimide, N,N'-carbonyldiimidazole, N,N'-thionyldiimidazole and diethyldiazodicarboxylate.

B. The compounds of the formula I with the definition as in A can be obtained by N-substitution of a compound of the formula

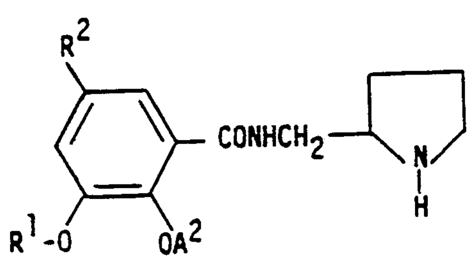

wherein $R^1$, $R^2$ and $A^2$ have the above given definition, with a compound of the formula

$$R^3—CH_2—X$$

wherein $R^3$ has the above given definition and X is a leaving group such as chlorine, bromine, sulphate, phosphate, benzenesulphonate or toluenesulphonate.

The reaction can preferably be effected by treating the reactants at 20°C to reflux temperature in a suitable solvent, e.g. acetone, alcohols, dimethylformamide (DMF), dimethylsulphoxide (DMSO) in the presence of a base, for example NaOH or $K_2CO_3$.

Alternatively, the secondary amine can be N-substituted by reductive alkylation with an aldehyde of the formula

$R^3$—CHO

wherein $R^3$ has the above definition, in the presence of a reducing agent such as sodium cyanoborohydride, Raney nickel, boranes, $H_2$/Pd, $H_2$/Pt or a metal/acid system having a metal like zinc.

Alternatively, the secondary amine can be N-substituted by acylation with a reagent such as $R^3COCl$, $(R^3—CO)_2$ or $R^3$—COOMe to give

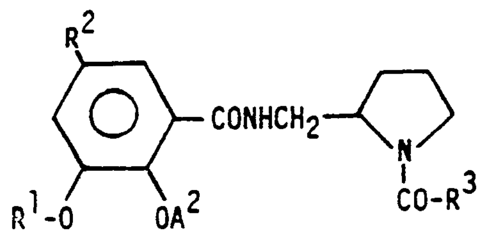

which is subsequently reduced with a reducing agent such as $LiAlH_4$ and alkoxy complexes thereof; $NaBH_4$ in pyridine or with addition of transition metal salts, or $AlCl_3$, or $BF_3$ or $POCl_3$ or carboxylic acids such as $CH_3COOH$ and $CF_3COOH$; $B_2H_6$. The reduction is preferably carried out in ether solvents such as diethylether, dimethoxyethane, diglyme, THF or dioxane at temperatures from 0°C to reflux.

C. The compounds of the formula $I^c$

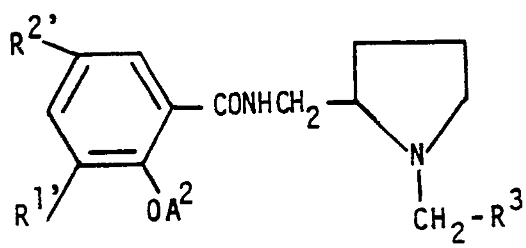

$I^c$

wherein $A^2$ and $R^3$ have the definitions given under A and $R^{1'}$ is a hydrogen atom or an alkyl(1—4C) group and $R^{2'}$ is a bromine or chlorine atom, or a physiologically acceptable salt or optical isomer thereof, can be obtained by reaction of a compound of the formula

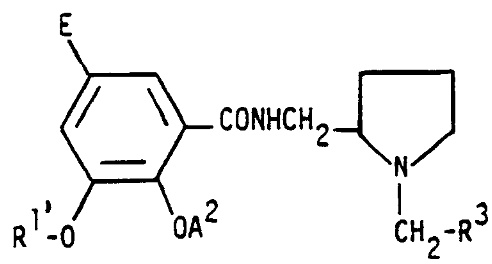

wherein $R^{1'}$, $A^2$ and $R^3$ have the definitions given above and E is hydrogen or $Si(CH_3)_3$, with a chlorinating or brominating reagent.

Chlorination is effected by treating the starting compound with sulphurylchloride or with chlorine with or without Lewis acid catalysis or with HOCl or with N-chloroamides in the presence of acid catalyst in suitable solvent, e.g. chloroform, nitrobenzene.

Bromination is carried out with $Br_2$ with or without Lewis acid catalysis or bromination in acetic acid in the presence of a base e.g. sodium acetate or by using bromine-dioxane complex. Other reagents can be used among them HOBr and N-bromoamides especially N-bromosuccinimide with acid catalysis.

D. The compounds of the formula $I^d$

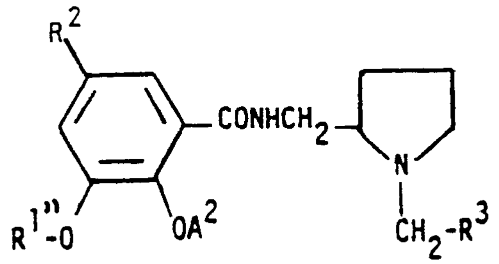

$I^d$

wherein $R^2$, $R^3$ and $A^2$ have the definitions given under A and $R^{1''}$ is an alkyl(1—4C) group, or a physiologically acceptable salt or optical isomer thereof, can be obtained by reaction of a compound of the formula

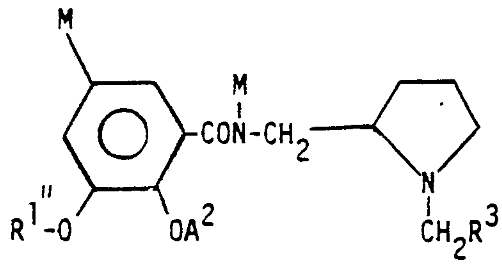

wherein $R^{1''}$, $R^3$ and $A^2$ have the definitions given above and M is a metal derivative, with an electrophilic reagent. The metal derivative M can be for instance Li or MgBr.

Suitable electrophilic reagent is for instance $Br_2$, $I_2$, hexachloroethane, alkyl iodide, trifluoroalkyl iodide, dialkyl disulphide or trimethylchlorosilane.

E. The compound of the formula $I^{ef}$

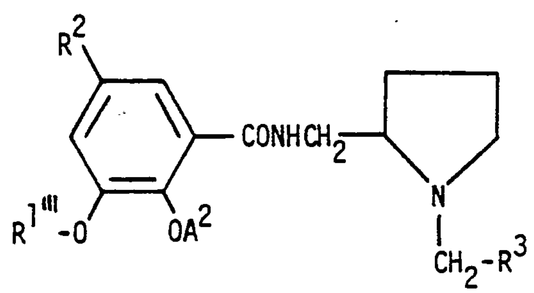

$I^{ef}$

wherein $R^2$, $R^3$ and $A^2$ have the definitions given under A and $R^{1'''}$ is an alkyl(1—4C) group, and alkyl(1—20C)—CO group, an alkyl(1—20C)—O—CO group, an alkyl(1—20C)—NH—CO group or an

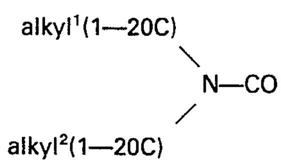

group, or a physiologically acceptable salt or optical isomer thereof, can be obtained by O-alkylation of a compound of the formula

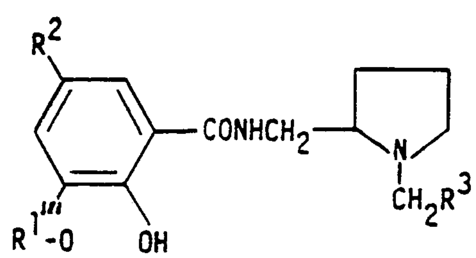

wherein $R^{1'''}$, $R^2$ and $R^3$ have the definitions given above with a compound of the formula

$$A^2—B$$

wherein $A^2$ has the definition given above and B is a leaving group.

The leaving group B can be for instance $—(SO_4)_{1/2}$, $—(PO_4)_{1/3}$ or a halogen.

The reaction is effected in a suitable solvent, e.g. acetone or DMF preferably at elevated temperatures with one equivalent of alkylating agent in the presence of base, e.g. alkali metal carbonates or hydroxides.

F. The compound of the formula $I^{ef}$

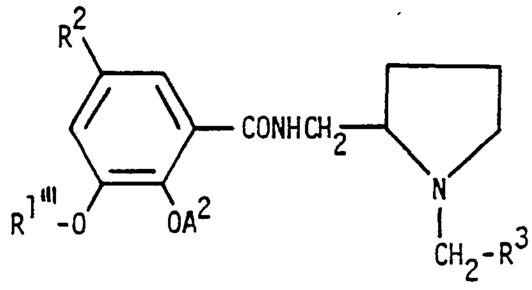

$I^{ef}$

wherein $R^2$, $R^3$ and $A^2$ have the definitions given under A and $R^{1'''}$ is an alkyl(1—4C) group, and alkyl(1—20C)—CO group, an alkyl(1—20C)—O—CO group, and alkyl(1—20C)—NH—CO group or an

alkyl¹(1—20C)
\
N—CO
/
alkyl²(1—20C)

group, or a physiologically acceptable salt or optical isomer thereof, can be obtained by reaction of a compound of the formula

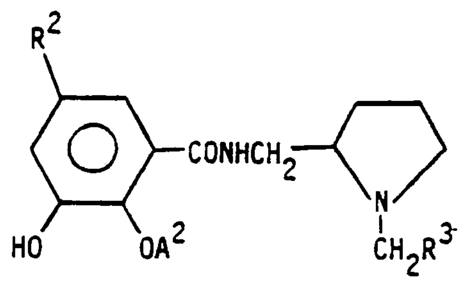

wherein $R^2$, $R^3$ and $A^2$ have the definitions given above with a compound of the formula

$$R^{1'''}—B$$

wherein $R^{1'''}$ has the definition given above and B is a leaving group.

The leaving group B can be as defined in E.

The reaction is performed in a suitable solvent preferably in the presence of a base such as a tertiary amine or alkali metal carbonate.

Alternatively, the reaction can be done with an isocyanate R—N=C=O in the case of $R^1$ being monoalkylcarbamoyl.

Intermediates

The compounds of the formula

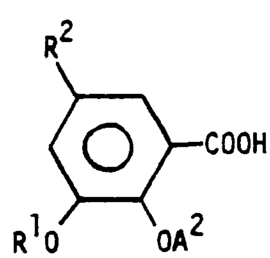

wherein

$R^1$ is a hydrogen atom, an alkyl group, an acyl group, an alkoxycarbonyl group or a mono- or dialkylcarbamoyl group;

$R^2$ is a halogen atom, an alkyl group, a trifluoroalkyl group, an alkylthio group or a trimethylsilyl group;

$A^2$ is a methyl or ethyl group,

provided that

a) when $A^2$ is ethyl, $R^2$ is a halogen atom, an alkyl group, a trifluoroalkyl group, an alkylthio group or a trimethylsilyl group;

b) when $A^2$ is methyl and $R^1$ is alkyl, $R^2$ is iodo, $C_2$—$C_4$ alkyl, a trifluoroalkyl group or a trimethylsilyl group,

are valuable intermediates for the preparation of the compounds of the formula I.

These compounds can be prepared according to the following reaction scheme

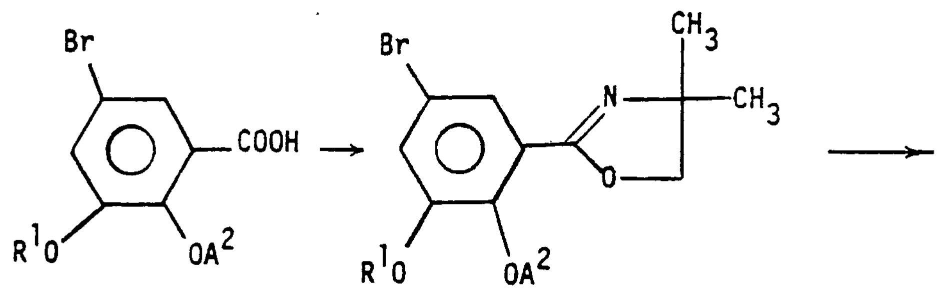

$R^1$ = H, alkyl)

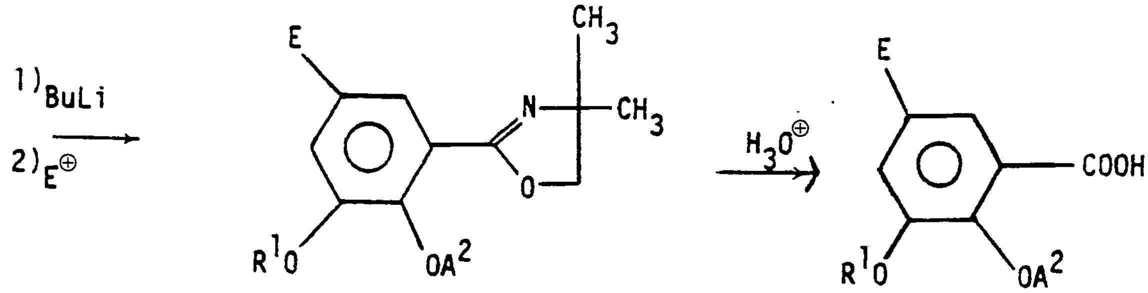


wherein E is Cl, I, F, Me, Et, Pr, Bu, $CF_3$-alkyl, S-alkyl or $Me_3Si$.

The preferred intermediates have $R^2$ = ethyl.

## Working examples

### Example 1

(S)-(−)-5-Bromo-N-[(1-ethyl-2-pyrrolidinyl)methyl]-2,3-dimethoxybenzamide (Method A)

To a solution of 5-bromo-2,3-dimethoxybenzoic acid (4.3 g, 0.016 mol, cf. Pettit and Piatak, J. Org. Chem. *25*, 721 (1960) and 0.4 ml dimethylformamide as catalyst in 45 ml toluene, thionyl chloride (4.5 g, 0.038 mol) was added at room temperature. The mixture was heated to 65°C for 1 h. After cooling the solvent was evaporated *in vacuo* and the residue consisting of 5-bromo-2,3-dimethoxybenzoyl chloride was dissolved in 25 ml of dichloromethane. A solution of (S)-(−)-2-aminomethyl-1-ethylpyrrolidine (2.6 g, 0.020 mol) in 50 ml $CH_2Cl_2$ was added and the mixture was stirred overnight at room temperature. The solvent was evaporated and the residue was portioned between 1 M NaOH and $Et_2O$. The organic layer was extracted three times with 1 M HCl and the aqueous phase was made alkaline and extracted three times with $CH_2Cl_2$. Drying and evaporation gave 5.5 g (93%) pure amide as an oil. $^1H$ NMR($CDCl_3$) δ7.85 (d, 1, J=2.4Hz, 6-H), 7.13 (d, 1, J, =2.4Hz, 4-H), 3.89 (s, 6, 2,3-$(OMe)_2$); $^{13}C$ NMR ($CDCl_3$) δ164.0, 153.4, 146.9, 128.2, 125.6, 118.3, 116.9, 62.3, 61.3, 56.4, 53.5, 47.8, 41.1, 28.4, 22.7, 14.0: $[\alpha]_D^{25}$ −59° (c=1.15 (acetone); MS (EI, 70 eV); m/z (rel. Int.) 372/370 M, 0.07%/0.08%), 245/243 (5-Br-2,3-$(OMe)_2C_6H_2CO$, 0.98%/1.04%), 98 (100%).

The hydrobromide of the title compound was precipitated from acetone/EtOH/$Et_2O$ and recrystallized from EtOAc to give 5.0 g (69%), mp. 135—136°C; $[\alpha]_D^{25}$ −12.5° (c=1.04, $H_2O$);

Anal. calcd. for $C_{16}H_{23}BrN_2O_3 \cdot HBr$: C, 42.50; H, 5.35; Br, 35.34; N, 6.20.

Found: C, 42.47; H, 5.29; Br, 35.21; N, 6.22.

### Example 2

(R)-(+)-5-Bromo.N.[(1.ethyl-2-pyrrolidinyl)methyl]-2,3-dimethoxybenzamide (Method A)

5-Bromo-2,3-dimethoxybenzoyl chloride (10 mmol) was reacted with (R)-(+)-2-aminomethyl-1-ethyl-

pyrrolidine (11 mol) in 25 ml $CH_2Cl_2$ overnight. Work up as above gave 3.72 g (100%) pure amide. $[\alpha]_D^{22}$ + 61° (c=1.27, acetone).

The hydrobromide was prepared and recrystallized from EtOH/$Et_2O$ to give 3.92 g (87%), mp. 135—136°C.

Anal. calcd. for $C_{16}H_{23}BrN_2O_3 \cdot HBr$: C, 42.50; H, 5.35; Br, 35.34; N, 6.20.
Found: C, 42.48; H, 5.31; Br, 35.56; N, 6.20.

Example 3

(S)-(−)-N-[(1-Ethyl-2-pyrrolidinyl)methyl]-2,3-dimethoxy-5-methyl-benzamide (Method A)

A mixture of 2,3-dimethoxy-5-methylbenzoic acid (0.30, 1.53 mmol), thionyl chloride (0.20 ml, 2.8 mmol) and dimethylformamide (3 drops) was stirred in 10 ml toluene at 60°C under $N_2$ for 1 h. The solvent was evaporated and the residue dissolved in 10 ml $CH_2Cl_2$ and evaporated again. The residue was dissolved in 10 ml $CH_2Cl_2$ and a solution of (S)-(−)-2-aminomethyl-1-ethyl-pyrrolidine (0.78 g, 1.83 mmol) in 5 ml $CH_2Cl_2$ was added. The mixture was stirred overnight at room temperature and then evaporated. The residue was dissolved in 2M HCl and washed with $Et_2O$. The aqueous layer was made alkaline and extracted twice with $Et_2O$. Drying ($MgSO_4$) and evaporation gave 420 mg (90%) of title compound as an oil. $^1H$ NMR ($CDCl_3$) δ7.51 (d, 1, 6-H), 6.85 (d, 1, 4-H), 3.88 (s, 6, 2,3-$(OMe)_2$); $^{13}C$ NMR ($CDCl_3$) δ165.5, 152.2, 145.5, 133.9, 126.3, 122.8, 116.1, 62.5, 61.1, 56.9, 53.4, 47.9, 41.1, 28.3, 22.5, 21.1, 13.6; MS (EI, 70 eV): m/z (rel. int.) 179 (5-Me-2,3-$(OMe)_2C_6H_2CO$, 4.2%). 98 (100%); $[\alpha]_D^{22}$ −73° (c=1.9, acetone).

Anal. calcd. for $C_{17}H_{26}N_2O_3$: C, 66.64; H, 8.55; N, 9.14.
Found: C, 66.71; H, 8.50; N, 8.52.

Example 4

Analogously the following compounds were prepared (Method A):

a) (S)-(−)-N-[(1-Ethyl-2-pyrrolidinyl)methyl]-2,3-dimethoxy-5-ethylbenzamide

Yield: 55% of an oil. $^1H$ NMR ($CDCl_3$) δ7.57 (d, 1, 6-H), 6.88 (d, 1, 4-H), 3.89 and 3.88 (two s, 6, 2,3-$(OMe)_2$; $^{13}C$ NMR ($CDCl_3$) δ165.5, 152.3, 145.6, 140.2, 126.4, 121.7, 114.9, 62.4, 61—1. 56.0, 53.4, 47.8, 41.1, 28.6, 28.4, 22.5, 15.3, 13.8; MS (EI, 70 eV): m/z (rel. int.) 193 (5-Et2,3-$(OMe)_2C_6H_2CO$, 5.1%) 98 (100%); $[\alpha]_D^{22}$ −64° (c=0.59, acetone).

Anal. calcd. for $C_{18}H_{28}N_2O_3$: C, 67.47; H, 8.81; N, 8.74.
Found: C, 66.43; H, 8.59; N, 8.54.

b) (S)-(−)-N-[(1-Ethyl-2-pyrrolidinyl)methyl]-5-chloro-2,3-dimethoxybenzamide

Yield: 91% of an oil which solidified, mp. 50—52°C. $^1H$ NMR ($CDCl_3$) 7.76 (d, 1, 6-H), 7.03 (d, 1, 4-H), 3.89 (s, 6, 2,3-$OMe)_2$); $^{13}C$ NMR ($CDCl_3$) δ163.9, 153.1, 146.3, 129.3, 127.7, 122.2, 115.3, 62.2, 61.2, 56.2, 53.3, 47.7, 40.9, 28.2, 22.5, 13.7; MS (EI, 70 eV): m/z (rel. int.) 201/199 (5-Cl-2,3-$(OMe)_2C_6H_2CO$, 1.4%/4.1%, 98 (100%); $[\alpha]_D^{22}$ −72° (c=7.5, acetone).

Anal. calcd. for $C_{16}H_{23}ClN_2O_3$: C, 58.80; H, 7.09; N, 8.57.
Found: C, 58.77; H, 7.18; N, 8.43.

c) (S)-(−)-N-[(1-Ethyl-2-pyrrolidinyl)methyl]-2,3-dimethoxy-5-methyl-thiobenzamide

Yield: 94% of an oil. $^1H$ NMR ($CDCl_3$) δ7.67 (d, 1, 6-H), 7.00 (d, 1, 4-H), 3.89 (s, 6, 2,3-$(OMe)_2$); $^{13}C$ NMR ($CDCl_3$) δ164.8, 152.5, 145.3, 134.1, 126.8, 119.8, 113.8, 62.2, 61.1, 56.0, 53.3, 47.7, 40.9, 28.2, 22.4, 16.1, 13.7; MS (EI, 70 eV): m/z (rel. int.) 211 (5-MeS-2,3-$(OMe)_2$-$C_6H_4CO$, 5.1%), 98 (100%); $[\alpha]_D^{22}$ −80° (c=4.1, acetone).

Anal. calcd. for $C_{17}H_{26}N_2O_3S$: C, 60.33, H, 7.74; N, 8.28.
Found: C, 60.22; H, 7.83; N, 8.18.

d) (S)-(−)-N-[(1-Ethyl-2-pyrrolidinyl)methyl]-5-bromo-3-hydroxy-2-methoxybenzamide

Yield: 80% of an oil which solidified, m.p. 93—95°C. $^1H$ NMR ($CDCl_3$) δ8.88 (d, 1, 6-H), 7.17 (d, 1, 4-H), 3.88 (s, 3, OMe); $^{13}C$ NMR ($CDCl_3$) δ165.5, 152.2, 146.2, 127.4, 123.5, 123.3, 116.8, 62.7, 61.0, 53.4, 48.2, 41.4, 28.4, 22.5, 13.3; $[\alpha]_D^{22}$ −59° (c=0.34, acetone). MS (EI, 70 eV): m/z (rel. int.) 358/356 (M, 0.03%/0.04%), 231/229 (5-Br-3-(OH)-2-$(OCH_3)C_6H_2CO$, 0.97%/1.08%), 98 (100%).

e) (S)-(−)-N-[(1-Ethyl-2-pyrrolidinyl)methyl]-2,3-dimethoxy-5-butyl-benzamide

Yield: 96% of an oil. $^1H$ NMR ($CDCl_3$) δ7.55 (d, 1, 6-H), 6.86 (d, 1, 4-H), 3.89 and 3.88 (two s, 6, 2,3-$(OMe)_2$; $^{13}C$ NMR ($CDCl_3$) δ165.6, 152.3, 145.5, 139.1, 126.3, 122.3, 115.3, 62.4, 61.1, 56.0, 53.5, 47.8, 41.1, 35.5, 33.5, 28.4, 22.8, 22.6, 22.3, 13.9; MS (EI, 70 eV): m/z (rel. int.) 348 (M, 0.08%), 221 (5-Bu-2,3-$(OMe)_2C_6H_2CO$, 2.5%), 98 (100%); $[\alpha]_D^{22}$ −57° (c=0.47, acetone).

Example 5

(R)-(+)-5-Bromo-N-[(1-(4-fluorobenzyl)-2-pyrrolidinyl)methyl]-2,3-dimethoxybenzamide (Method A)

5-Bromo-2,3-dimethoxybenzoyl chloride (1.2 mmol) was reacted with (2R)-1-(4-fluorobenzyl)-2-aminomethylpyrrolidine (1.1 mmol) in 18 ml dichloromethane overnight. After evaporation the residue was partitioned between 1.5 M HCl and $Et_2O$/EtOAc. The aqueous phase was made alkaline and extracted with

$CH_2Cl_2$. Drying ($MgSO_4$) and evaporation gave 385 mg (78%) pure amine. $^1H$ NMR ($CDCl_3$) δ7.97 (d, 6-H), 6.9—7.6 (m), 3.95 and 3.91 (two s, 2,3-$(OMe)_2$); $[\alpha]_D^{22}$ +85° (c=0.96, acetone).

The hydrochloride salt was precipitated from EtOH/$Et_2O$ and recrystallized from acetone to give 340 mg, mp. 157.5—159°C.

Example 6

(S)-(−)-N-[(1-Allyl-2-pyrrolidinyl)methyl]-5-bromo-2,3-dimethoxybenzamide (Method B)

a) (S)-(+)-5-Bromo-N-(2-pyrrolidinylmethyl)-2,3-dimethoxybenzamide

5-Bromo-2,3-dimethoxybenzoyl chloride (3.8 mmol) was reacted with (S)-(−)-1-trityl-2-aminomethylpyrrolidine (1.35 g, 3.9 mmol) in 10 ml dichloromethane at room temperature for 1 h. The solvent was evaporated and the residue was treated with 10 ml EtOH and 0.1 ml conc. HCl for 1 h at room temperature. After evaporation the residue was partitioned between 0.5 M HCl and $Et_2O$. The aqueous phase was made alkaline, extracted with dichloromethane, dried ($Na_2SO_4$) and evaporated to give 1.10 g (84%) title compound as an oil. $^1H$ NMR ($CDCl_3$) δ7.93 (d, 1, 6-H), 7.23 (d, 1, 4-H), 3.94 (s, 6, 2,3-$(OMe)_2$); MS (EI, 70 eV): m/z (rel. int.) 342/340 (M-H, 0.20%/0.19%), 245/243 (5-Br-$(OMe)_2C_6H_2CO$, 4.5%/4.8%), 70 (100%); $[\alpha]_D^{22}$ +3.4° (c=0.42, acetone).

b) (S)-(−)-N-[(1-Allyl-2-pyrrolidinyl)methyl]-5-bromo-2,3-dimethoxybenzamide

Allyl bromide (105 µl, 1.25 mmol) was added to a mixture of (S)-(+)-5-bromo-N-(2-pyrrolidinylmethyl)-2,3-dimethoxybenzamide (400 mg, 1.17 mmol), potassium carbonate (200 mg, 1.45 mmol) and 10 ml dimethylformamide. After stirring for 1.5 h at room temperature the mixture was partitioned between 50 ml 0.2 M HCl and 50 ml ether. The aqueous phase was made alkaline and extracted twice with ether. Drying ($Na_2SO_4$) and evaporation gave a residue which was purified by flash chromatography on $SiO_2$ with i-$Pr_2O$/MeOH/$NH_3$ 100:10:1 as eluent to afford 370 mg (83%) pure title compound. $^1H$ NMR ($CDCl_3$) δ7.91 (d, 1, 6-H), 7.18 (d, 1, 4-H), 5.0—6.05 (m, 3, CH=$CH_2$), 3.91 (s, 6, $(OMe)_2$); MS (EI, 70 eV): m/z (rel. int.) 384/382 (M, 0.28%/0.28%), 245/243 (5-Br-2,3-$(OMe)_2C_5H_2CO$, 1.3%/1.4%), 110 (100%); $[\alpha]_D^{22}$ −57° (c=1.06, acetone).

Example 7

(S)-(−)-5-Bromo-N-[(1-(4-fluorobenzyl)-2-pyrrolidinyl)-methyl]-2,3-dimethoxybenzamide

The title compound was prepared according to example 6 in 84% yield as a hydrochloride monohydrate salt, mp. 157—159°C (recrystallized from acetone). $[\alpha]_D^{22}$ −10.1° (c=0.54, MeOH); MS (EI, 70 eV): m/z (rel. int.) 452/450/448 (M and M-H, 0.09%/0.18%/0.12%), 245/243 (5-Br-2,3-($OMe_2$-$C_6H_2CO$, 2.3%/2.4%), 178 (100%), 109 (90%).

Anal. calcd. for $C_{21}H_{24}Br\text{-}FN_2O_3 \cdot HCl \cdot H_2O$: C, 49.87; H, 5.38; N, 5.53.

Found: C, 49.90; H, 5.21; N, 5.48.

Example 8

(S)-(−)-5-Bromo-N-[(1-(4-fluorobenzyl)-2-pyrrolidinyl)-methyl-2,3-dimethoxy-5-iodobenzamide (Method D)

A solution of (S)-(−)-N-[(1-ethyl-2-pyrrolidinyl)methyl]-2,3-dimethoxy-5-bromobenzamide (245 mg, 0.68 mmol) in 5 ml tetrahydrofuran was added to a mixture of potassium hydride (39 mg, 0.97 mmol. oil dispersion washed with hexane and evaporated under $N_2$) and 5 ml tetrahydrofuran at −20°C under a nitrogen atmosphere. The temperature was allowed to gradually reach room temperature and after 1 h the mixture was cooled to −78°C and butyllithium (0.63 ml of 1.5 M hexane solution, 0.95 mmol) was added dropwise. After stirring for 1.5 h at −78°C a solution of iodine (500 mg, 2.0 mmol) in 3 ml tetrahydrofuran was added rapidly and the temperature was raised to room temperature over 0.5 h. After 0.5 h 50 ml 0.5 M HCl was added and the mixture extracted three times with ether. The aqueous layer was made alkaline and extracted with dichloromethane, dried ($Na_2SO_4$) and evaporated to give a crude oil containing 34% title compound and 45% of the hydrolysis product N-[(1-ethyl-2-pyrrolidinyl)-methyl]-2,3-dimethoxybenzamide. Repeated radial disc chromatography with i-$Pr_2O$/MeOH/$NH_3$ 100:5:1 as eluent gave 70 mg (25%) pure title compound. $^1H$ NMR ($CDCl_3$) δ8.12 (d, 1, 6-H), 7.37 (d, 1, 4-H), 3.91 (s, 6, $(OMe)_2$); MS (EI, 70 eV); m/z (rel. int.) 418 (M, 0.05%), 291 (5-I-2,3-$(OMe)_2C_6H_2CO$, 1.8%), 165 (0.28%), 164 (0.22%), 111 (2.0%), 98 (100%); $[\alpha]_D^{22}$ −48° (c=1.40, acetone).

Anal. calcd. for: $C_{16}H_{23}IN_2O_3$: C, 45.95; H, 5.54; N, 6.70.

Found: C, 46.05; H, 5.63; N, 6.64.

Example 9

Intermediary compounds

a) N-(3-Hydroxy-2-methylpropyl)-5-bromo-2,3-dimethoxybenzamide

A mixture of 5-bromo-2,3-dimethoxybenzoic acid (15.0 g, 57 mmol), thionyl chloride (12.8 ml, 172 mmol) in 100 ml toluene was heated at 50°C for 2 h. The solvent was evaporated and $CH_2Cl_2$ was added to the residue and then evaporated. The acid chloride was dissolved in 50 ml $CH_2Cl_2$ and a solution of 2-amino-2-methyl-1-propanol (10.3 g, 115 mmol) in 50 ml $CH_2Cl_2$ was added at +10°C over 10 min. After stirring at room temperature for 2 h another 200 ml $CH_2Cl_2$ was added and the mixture washed with water, dried ($Na_2SO_4$) and evaporated to give 19.0 g. Flash chromatography on $SiO_2$ with $iPr_2O$ as eluent afforded 14.7 g (77%) pure amide. $^1H$ NMR ($CDCl_3$) δ8.26 (br, 1, NH), 7.86 (d, 1, 6-H), 7.22 (d, 1, 4-H), 4.76 (t, 1, OH),

3.92 (s, 6, OMe), 3.72 (d, 2, $CH_2O$), 1.40 (s, 6, $CH_3$).

b) 2-(5-Bromo-2,3-dimethoxyphenyl)-4,4-dimethyl-2-oxazoline

N-(3-Hydroxy-2-methylpropyl)-5-bromo-2,3-dimethoxybenzamide (13.0 g, 39 mmol) was cyclized by dropwise addition of thionyl chloride (6.6 g, 55 mmol) at room temperature. After stirring for 0.5 h the mixture was poured into $Et_2O$ and 1 M NaOH was added. The ethereal layer was separated, dried ($Na_2SO_4$) and evaporated to give 10.0 g (82%) pure oxazoline. $^1H$ NMR ($CDCl_3$) δ7.54 (d, 1, 6-H), 7.15 (d, 1, 4-H), 4.12 (s, 2, $CH_2$), 3.86 and 3.85 (two s, 6, OMe), 1.37 (s, 6, $C(CH_3)_2$).

c) 2-(2,3-Dimethoxy-5-methyl)-4,4-dimethyl-2-oxazoline

To a solution of 2-(5-Bromo-2,3-dimethoxyphenyl)-4,4-dimethyl-2-oxazoline (2.0 g, 6.4 mmol) in 20 ml anhydrous tetrahydrofuran was injected 4.38 ml of 1.6 M butyllithium in hexane (7.0 mmol) at −78°C under $N_2$. After stirring for 1 h methyl iodide (1.99 g, 14 mmol) was injected and the temperature was raised to −45°C. After stirring another 1.5 h the mixture was poured into 100 ml 1 M NaOH and extracted twice with $Et_2O$. The ethereal layer was dried ($Na_2SO_4$), partly concentrated in vacuo, filtered through a silica plug and evaporated to give 1.2 (76%) of an oil. $^1H$ NMR ($CDCl_3$) δ7.19 (d, 1, 6-H), 6.86 (d, 1, 4-H), 4.13 (s, 2, $CH_2$), 3.86 (s, 5, OMe), 2.30 (s, 3, $CH_3$), 1.37 (s, 6, $C(CH_3)_2$).

The following compounds were prepared analogously by reaction of 2-(2,3-dimethoxy-5-lithiophenyl)-4,4-dimethyl-2-oxazoline and the indicated electrophile:

2-(5-Ethyl-2,3-dimethoxyphenyl)-4,4-dimethyl-2-oxazoline

Reaction with 2.1 equivalents of ethyl iodide. Separation on $SiO_2$ column with $iPr_2O$ as eluent afforded 67% pure oxazoline. $^1H$ NMR ($CDCl_3$) δ7.22 (d), 6.91 (d), 4.13 (s), 3.87 and 3.85 (two s), 2.64 (q, 2, $CH_2CH_3$), 1.37 (s), 1.23 (t, 3, $CH_2CH_3$).

2-(5-Butyl-2,3-dimethoxyphenyl)-4,4-dimethyl-2-oxazoline

Reaction with 1.5 equivalents of butyl bromide. Separation on $SiO_2$ column with $iPr_2O$ as eluent gave 81% oxazoline. $^1H$ NMR ($CDCl_3$) δ7.21 (d), 6.88 (d), 4.13 (s), 3.87 and 3.85 (two s), 2.60 (t), 1.4 (m), 0.92 (t).

2-(5-Chloro-2,3-dimethoxyphenyl)-4,4-dimethyl-2-oxazoline

Reaction with 1.6 equivalents of hexachloroethane. Separation on $SiO_2$ column with $iPr_2O$ as eluent afforded 49% oxazoline. $^1H$ NMR ($CDCl_3$) δ7.38 (d), 7.00 (d), 4.13 (s), 3.88 and 3.85 (two s), 1.37 (s).

2-(2,3-Dimethoxy-5-methylthiophenyl)-4,4-dimethyl-2-oxazoline

Reaction with 1.6 equivalents of dimethyl disulfide. Separation on $SiO_2$ column with $iPr_2O$ as eluent afforded 44% oxazoline. $^1H$ NMR ($CDCl_3$) δ7.38 (d), 7.00 (d), 4.13 (s), 3.89 and 3.86 (two s), 2.50 (s, 3, $SCH_3$), 1.39 (s).

d) 2,3-Dimethoxy-5-methylbenzoic acid

2-(2,3-Dimethoxy-5-methylphenyl)-4,4-dimethyl-2-oxazoline (1.1 g, 4.4 mmol) was heated to reflux in 10 ml 2 M HCl for 1 h. The mixture was extracted with $Et_2O$. Drying ($Na_2SO_4$) and evaporation gave 0.55 g (63%) crystalline acid, which was recrystallized from $iPr_2O$ to give 0.40 g. mp. 92—93°C. $^1H$ NMR ($CDCl_3$) δ7.48 (d, 1, 6-H), 6.97 (d, 1, 4-H), 4.03 (s, 3, 2-OMe), 3.90 (s, 3, 3-OMe), 2.34 (s, 3, $CH_3$); $^{13}C$ NMR ($CDCl_3$) δ165.9, 151.9, 146.3, 134.9, 123.8, 121.7, 118.6, 62.1, 56.2, 21.1;

Anal. calcd. for $C_{10}H_{12}O_4$: C, 61.22; H, 6.17; O, 32.62.

Found: C, 61.05: H, 6.05; O, 32.70.

The following compounds were prepared analogously:

5-Ethyl-2,3-Dimethoxybenzoic acid

Yield: 70% of an oil. $^1H$ NMR ($CDCl_3$) δ10.2 (br), 7.53 (d), 700 (d), 4.05 (s), 3.93 (s), 2.65 (q), 1.25 (t); MS (EI, 70 eV): m/z (rel. int.) 210 (M, 100%), 195 (M-$CH_3$, 86%), 177 (29%), 165 (21%), 163 (41%).

5-Butyl-2,3-dimethoxybenzoic acid

Yield: 52% of an oil. $^1H$ NMR ($CDCl_3$) δ10.3 (br), 7.58 (d), 7.05 (d), 4.07 (s), 3.95 (s), 2.65 (t), 1.1—2.0 (m), 0.94 (s).

5-Chloro-2,3-dimethoxybenzoic acid

Yield: 83%. Mp. 124—126°C ($iPr_2O$/hexane). $^1H$ NMR ($CDCl_3$) δ10.4 (br), 7.68 (d), 7.17 (d), 4.05 (s), 3.94 (s).

Anal. calcd. for $C_9H_9ClO_4$: C, 49.90; H, 4.19; Cl, 16.37; O, 29.54.

Found: C, 49.76; H, 4.23; Cl, 16.19; O, 29.69.

2,3-Dimethoxy-5-methylthiobenzoic acid

Yield: 76%. Mp. 90—92°C ($iPr_2O$/hexane). $^1H$ NMR ($CDCl_3$) δ10.2 (br), 7.55 (d), 7.08 (d), 4.05 (s), 3.92 (s), 2.52 (s).

Anal. calcd. for $C_{10}H_{12}O_4S$: C, 52.61; H, 5.30; O, 28.03; S, 14.05.
Found: C, 52.57; H, 5.27; O, 28.13; S, 14.13.

e) 5-Bromo-3-hydroxy-2-methoxybenzoic acid

To a mixture of 2,3-dihydroxybenzoic acid (12 g, 0.078 mol) and $K_2CO_3$ (32 g, 0.23 mol) in 150 ml dimethylformamide benzoyl chloride (23 g, 0.16 mol) was added dropwise. The reaction mixture was heated for 8 h at 80°C and then poured into water. The precipitate was recrystallized from acetone/water (3:7) to give 12.2 g (61%) of 3-benzoyloxy-2-hydroxybenzoic acid, mp. 194—196°C.

Bromine (5.8 g, 0.036 mol) was added dropwise to a suspension of the above acid (7.7 g, 0.03 mol) and sodium acetate (3.0 g, 0.036 mol). After stirring for 3 h at 70°C the mixture was poured into ice-water. The solid was recrystallized from $CHCl_3$/hexane to give 8.1 g (80%) of 3-benzoyloxy-5-bromo-2-hydroxybenzoic acid, mp. 172—174°C.

The above bromo acid (6.7 g, 0.02 mol) was dissolved in 100 ml dimethyl sulfoxide and sodium hydride (suspension in mineral oil, 0.05 mol) was added in portions under cooling in a $N_2$ atmosphere. After stirring at room temperature for 2 h methyl iodide (7.6 g, 0.06 mol) was added and the mixture was heated at 60°C for 2 h. The mixture was poured into ice-water, extracted with $Et_2O$ and evaporated. The diester obtained was hydrolyzed by reflux in aqueous potassium hydroxide acidified and extracted with EtOAc. The crude title compound was purified by chromatography on $SiO_2$ with EtOAc/HOAc (98:2) and recrystallized from $iPr_2O$/hexane to give 1.0 g (20%) pure 5-bromo-3-hydroxy-2-methoxybenzoic acid, mp. 121—123°C. $^1H$ NMR ($CDCl_3$) δ7.76 (d, 1, 6-H), 7.21 (d, 1, 4-H), 3.96 (s, 3, OMe).

Example 10

The following examples illustrate the preparation of pharmaceutical compositions of the invention. The wording "active substance" denotes a compound according to the present invention or a salt thereof, and preferably the compound N-ethyl-2-(3-bromo-2-hydroxy-6-methoxybenzamidomethyl)pyrrolidine or the 3-bromo-6-hydroxy-2-methoxy substituted isomer.

Formulation A. Soft gelatin capsules

500 g of active substance were mixed with 500 g of corn oil, whereupon the mixture was filled in soft gelatin capsules, each capsule containing 100 mg of the mixture (i.e. 50 mg of active substance).

Formulation B. Soft gelatin capsules

500 g of active substance were mixed with 750 g of peanut oil, whereupon the mixture was filled in soft gelatin capsules, each capsule containing 125 mg of the mixture (i.e. 50 mg of active substance).

Formulation C. Tablets

50 kg of active substance were mixed with 20 kg of silicic acid of the trademark Aerosil. 45 kg of potato starch and 50 kg of lactose were mixed therewith and the mixture was moistened with a starch paste prepared from 5 kg of potato starch and distilled water, whereupon the mixture was granulated through a sieve. The granulate was dried and sieved, whereupon 2 kg of magnesium stearate was mixed into it. Finally the mixture was pressed into tablets each weighing 172 mg.

Formulation D. Effervescing tablets

100 g of active substance, 140 g of finely divided citric acid, 100 g of finely divided sodium hydrogen carbonate, 3.5 g of magnesium stearate and flavouring agents (q.s.) were mixed and the mixture was pressed into tablets each containing 100 mg of active substance.

Formulation E. Sustained release tablet

200 g of active substance were melted together with 50 g of stearic acid and 50 g of carnauba wax. The mixture thus obtained was cooled and ground to a particle size of at most 1 mm in diameter. The mixture thus obtained was mixed with 5 g of magnesium stearate and pressed into tablets each weighing 305 mg. Each tablet thus contains 200 mg of active substance.

Formulation F. Injection solution

| | |
|---|---|
| Active substance | 3.000 mg |
| Sodium pyrosulfite | 0.500 mg |
| Disodium edetate | 0.100 mg |
| Sodium chloride | 8.500 mg |
| Sterile water for injection ad. | 1.00 ml |

Formulation G. Hard gelatine capsules

10 g of active substance was mixed with 400 g of lactose and finally 2 g of magnesium stearate was added. The mixture was then filled in hard gelatine capsules, each capsule containing 206 mg of the mixture (i.e. 5 mg of active substance).

Formulation H. Tablets

50 g of active substance was mixed with 1 500 g of lactose, 200 g of microcrystalline cellulose and 10 g magnesium stearate. Tablets of 5 mg active substance with a core weight of 176 mg were finally comprotted.

Formulation I. Depot preparation

| | |
|---|---|
| (S)-(−)-N-[(1-ethyl-2-pyrrolidinyl)-methyl]-5-bromo-2-methoxy-3-octa-decanoyloxy benzamide | 200 mg |
| Peanut oil | ad 1 ml |

Pharmacology

a) In vivo test: apomorphine syndrome inhibition

A number of studies suggest that the antipsychotic action of neuroleptic drugs is in some way related to the decrease in catecholamine transmission in the brain caused by these drugs and more specifically due to central dopamine (DA) receptor blockade in cortical and subcortical brain regions. Most compounds with an antipsychotic action effect several DA systems in the brain. There is evidence that the antipsychotic and cortical limbic structures (J. Pharm. Pharmacol. *25*, 346, 1973; Lancet, Nov. *6*, 1027, 1976) while the well-known extrapyramidal side effects produced by neuroleptic drugs are due to blockade of DA receptors in the nigroneostriatal DA system (Intern. J. Neurol. *6*, 27—45, 1967).

There are presently several techniques available to study DA receptor blockade in the brain *in vivo*. One method is based on the ability of antipsychotic drugs to block the behavioural effects induced by the DA agonist apomorphine in the rat. Several studies indicate an excellent correlation between the *in vivo* DA receptor blockades as measured in the apomorphine test and therapeutic efficacy of different antipsychotic drugs. Apomorphine produces in rats and other species a characteristic syndrome consisting of repetitive movements (stereotypies) and hyperactivity which appear to be due to activation of postsynaptic DA receptors in the brain (J. Pharm. Pharmacol. *19*, 627, 1967; J. Neurol. Transm. *40*, 97—113, 1977). The stereotypies (chewing, licking, biting) appear mainly to be induced via activation of DA receptors linked to the neostriatal DA system (J. Psychiat. Res., *11*, 1, 1974) whereas the increased locomotion (hyperactivity) mainly appears to be due to activation of DA receptors in mesolimbic structures (nucleus olfactorium, nucleus accumbens) i.e the mesolimbic DA system. (J. Pharm. Pharmacol. *25*, 1003, 1973).

A number of studies have demonstrated that neuroleptics of different structural classes block the apomorphine stereotypies in the rat and that this blockade is well related to blockade of DA transmission measured by biochemical or neurophysiological techniques. Thus, the antiapomorphine effect correlates well with changes in DA turnover produced by neuroleptic drugs (Eur. J. Pharmacol., *11*, 303, 1970), DA receptor binding studies (Life Science, *17*, 993—1002, 1976) and most important with antipsychotic efficacy (Nature, *263*, 388—341, 1976).

Methods

Male Sprague-Dawley rats (weighing 225—275 g) were used. The rats were observed in perspex cages (40 (L) × 25 (w) × 30 (h) cm) and the behaviour was scored 5, 20, 40, and 60 min. after apomorphine. The compounds were injected 60 min. prior to apomorphine hydrochloride (1 mg/kg) which was injected subcutaneously (s.c.) into the neck. This dose and form of administration was found to produce a very consistent response and very low variation in response strength. Furthermore, apomorphine given s.c. also produced a very consistent hyperactivity.

Directly after injection, the animals were placed in the cages, one in each cage. Scoring of the stereotypies were performed by two separate methods. The first scoring system was a modified version of the system introduced by Costall and Naylor (1973). The strength of the stereotype was scored on a 0—3 scale as follows:

| Score | Description of stereotyped behaviour |
|---|---|
| 0 | No change in behaviour compared to saline controls or sedated; |
| 1 | Discontinuous sniffing; |
| 2 | Continuous sniffing; |
| 3 | Continuous sniffing. Chewing, biting and licking. |

In the second system the number of animals displaying hyperactivity caused by apomorphine were scored. Each group consisted of 6—8 animals. Saline controls were always run simultaneously. $ED_{50}$'s are in the first scoring system (0—3 scale), the doses which reduce the strength of the stereotypes by 50% over the observation period of 60 min. $ED_{50}$'s of the second scoring system are the doses which reduce the number of animals showing hyperactivity by 50% over the observation period of 60 min. The $ED_{50}$ were calculated from log dose-response curves by the method of least squares from 4—6 dose levels with 6—8 animals per dose level.

a) In vitro test: Receptor binding assay

The clinical efficacy of antipsychotic drugs has been shown to correlate with their ability to displace tritiated spiperone from preparations of dopamine receptors (Seeman, Biochem. Pharmacol. *26*, 1741, (1977).

Method

The method of Burt *et al.* (Proc. Nat. Acad. Sci. USA *72*, 4655 (1975)) was used. Male Sprague-Dawley rats weighing 150—200 g were decapitated, and their brains were rapidly removed. The striata were dissected, pooled and homogenized in 50 mM Tris-HCl buffer (pH 7.6). The membrane fraction was collected by centrifugation (48 000 g for ten minutes), washed once with the buffer, and resuspended in 50 mM Tris HCl (pH 7.6) containing 0.1% ascorbic acid, 10 mM pargyline, 120 mM NACl, 5 mM KCl, 2 mM $CaCl_2$ and 1 mM $MgCl_2$. The suspension was preincubated at 37°C for 10 minutes and then kept on ice until use.

The assays have been carried out using a cell harvester equipment. The incubations were made in quadruplicate, each well containing membrane suspension (2.5 mg/0.5 ml), $^3$H-spiperone (0.4 nM) and the test compound in a final volume of 0.5 ml. After incubation for 10 minutes at 37°C, the contents of the wells were rapidly filtered and sashed on Whatman GF/B filters using the Cell harvester. The specific binding was defined as the difference of $^3$H-spiperone bound in the presence and in the absence of 1 μm (+)-butaclamol. The test results are expressed as IC50. The IC50 value given in nM, indicates the concentration of the test substance which reduces the amount of specifically bound $^3$H-spiperone by 50%.

Test results

The test results are given in the following table.

| Test compound | In vivo | | In vitro |
|---|---|---|---|
| | Reduction of stereotypies ED50 (µmole/kg i.p.) | Reduction of hyperactivity ED50 (µmole/kg i.p.) | Block of ³H-spiperone binding IC50 (nM) |
| Prior art compounds: 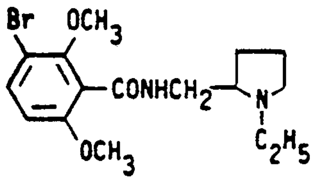 (remoxipride, US patent 4 232 037) | 6.5 | 0.86 | 1570 |
| 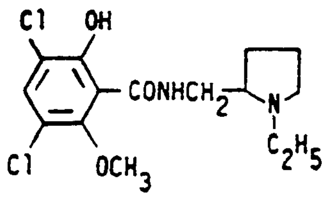 (a compound of EP 60235) | 2.4 | 0.11 | 26 |
| 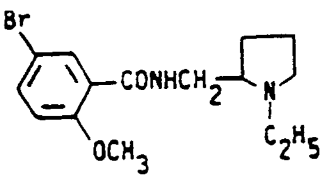 (ex 5 of US 3 342 826 | 6.5 | 2.5 | 44 |
| Compounds of the invention: | | | |
| 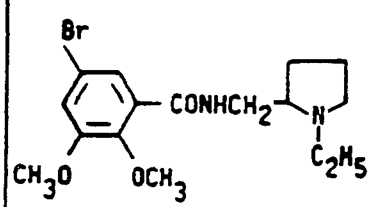 | 0.13 | 0.0032 | 1.2 |
| 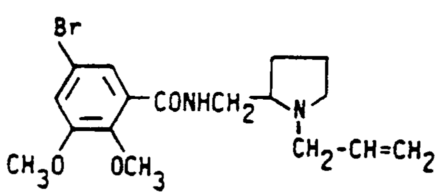 | 0.12 | 0.020 | 1.5 |

**Claims for the Contracting States: BE CH DE FR GB IT LI LU NL SE**

1. A compound of the formula

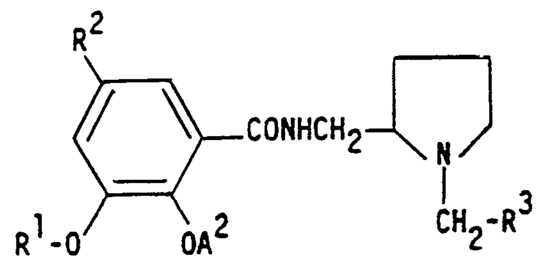

wherein

$R^1$ is a hydrogen atom, an alkyl (1—4C) group, an alkyl(1—20C)-CO group, an alkyl(1—20)-O—CO group, an alkyl(1—20)-NH—CO group or an

alkyl¹(1—20C)
\
N—CO group;
/
alkyl²(1—20C)

wherein alkyl¹ and alkyl² can be the same or different,

$R^2$ is a halogen atom, an alkyl(1—4C) group or an alkyl(1—4C)-thio group;

$A^2$ is a methyl or ethyl group; and

$R^3$ is a hydrogen atom, an alkyl(1—4C) group, an alkenyl(2—3C) group or a phenyl group substituted by one or more of fluoro, chloro, bromo, trifluoromethyl, ethyl, methoxy or ethoxy in the orto, meta or para positions, or substituted by methylenedioxy;

or a physiologically acceptable salt or optical isomer thereof.

2. A compound according to claim 1 with the formula I wherein

$R^1$ is hydrogen, methyl, ethyl or an alkyl(1—20C)-CO group;

$R^2$ is halogen or alkyl;

$A^2$ is methyl or ethyl; and

$R^3$ has the definition given in claim 1.

3. A compound according to claim 1 with the formula I wherein

$R^1$ is hydrogen, methyl or ethyl;

$R^2$ is chlorine, bromine, iodine, methyl, ethyl, n-propyl or n-butyl;

$A^2$ is methyl or ethyl; and

$R^3$ has the definition given in claim 1.

4. A compound according to claim 1 with the formula I wherein

$R^1$ is methyl;

$R^2$ is chlorine, bromine, iodine, methyl, ethyl, n-propyl or n-butyl;

$A^2$ is methyl; and

$R^3$ is a hydrogen atom, an alkyl(1—4C) group, an alkenyl(2—3C) group, or a phenyl group substituted in the para position with a fluorine atom.

5. A compound according to claim 1 with the formula I wherein

$R^1$ is methyl;

$R^2$ is bromine;

$A^2$ is methyl; and

$R^3$ has the definition given in claim 4.

6. A compound according to claim 1 with the formula

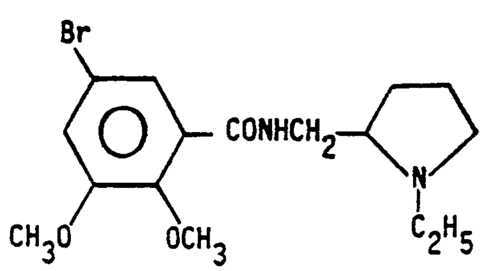 ; 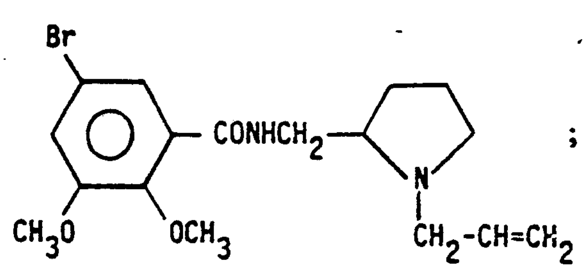 ;

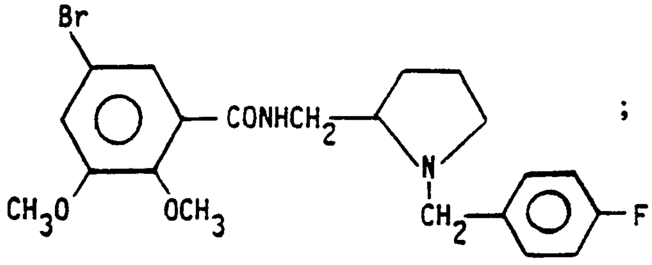 ; 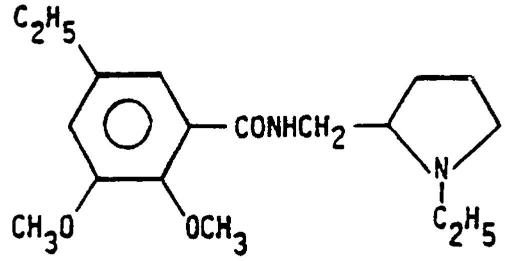 ;

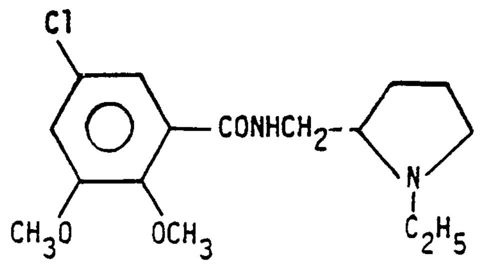 ; 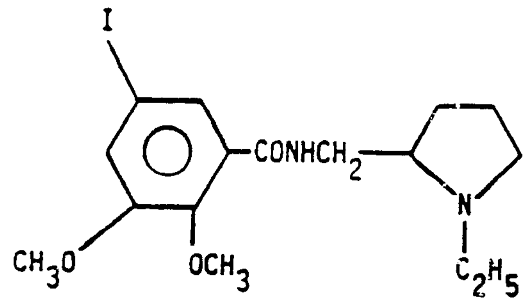 ;

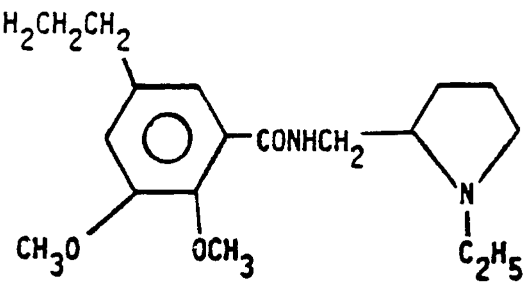

7. A compound according to any of claims 1—6 in the form of an optical isomer thereof.

8. A compound according to any of claims 1—7 in the form of a physiologically acceptable salt thereof.

9. A process for the preparation of a compound of the formula

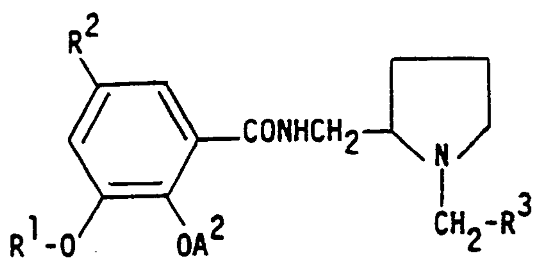 I

wherein

$R^1$, $R^2$, $R^3$ and $A^2$ have the definitions given in claim 1

or a physiologically acceptable salt or optical isomer thereof, which process comprises

a) reaction of a compound of the formula

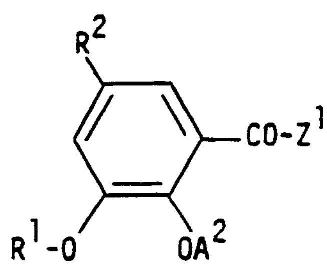

wherein $R^1$, $R^2$ and $A^2$ have the definitions given above and —CO—$Z^1$ is a reactive group capable of reacting

with an amino group under formation of an amide moiety, with a compound of the formula

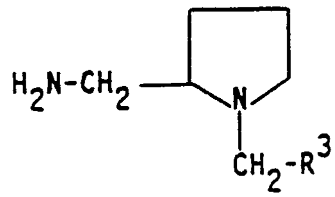

wherein $R^3$ has the definition given above, or a reactive derivative thereof, to the formation of a compound of the formula I or

b) N-substitution of a compound of the formula

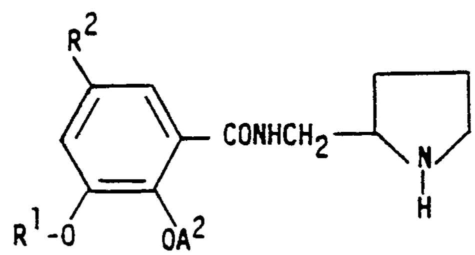

wherein $R^1$, $R^2$ and $A^2$ have the definitions given above with a compound of the formula

1) $R^3$—$CH_2$—X; or

2) $R^3CHO$ in the presence of a reducing agent; or

3) $R^3COCl$, $(R^3CO)_2O$ or $R^3$—$COOCH_3$ and subsequent reduction,

wherein $R^3$ has the definition given above and X is a leaving group, to the formation of a compound of the formula I, whereafter, if desired, the compound obtained by any of the methods a)—b) is converted to a physiologically acceptable salt thereof and/or converted to a substantially pure stereoisomer thereof.

10. A process for the preparation of a compound of the formula

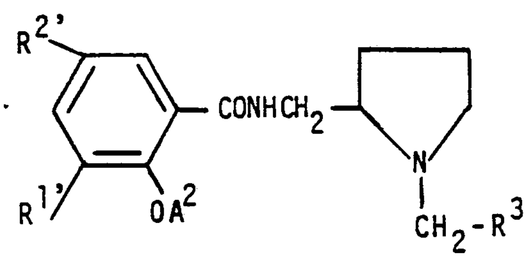

$I^c$

wherein $A^2$ and $R^2$ have the definitions given in claim 1 and $R^{1'}$ is a hydrogen atom or an alkyl(1—4C) group and $R^{2'}$ is a bromine or chlorine atom, or a physiologically acceptable salt or optical isomer thereof, which process comprises reaction of a compound of the formula

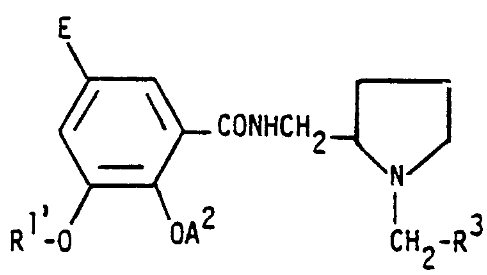

wherein $R^{1'}$, $A^2$ and $R^3$ have the definitions given above and E is hydrogen or $Si(CH_3)_3$, with a chlorinating or brominating reagent, to the formation of a compound of the formula $I^c$, whereafter, if desired, the compound obtained is converted to a physiologically acceptable salt thereof and/or converted to a substantially pure stereoisomer thereof.

11. A process for the preparation of a compound of the formula

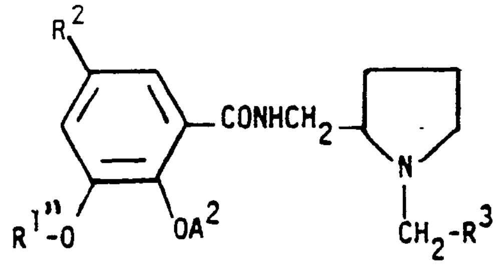

$I^d$

wherein $R^2$, $R^3$ and $A^2$ have the definitions given in claim 1 and $R^{1''}$ is an alkyl(1—4C) group, or a physiologically acceptable salt or optical isomer thereof, which process comprises reaction of a compound of the formula

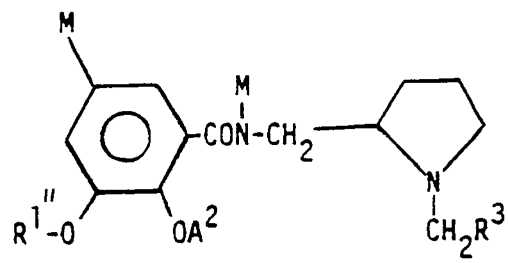

wherein $R^{1''}$, $R^3$ and $A^2$ have the definitions given above and M is a metal derivative, with an electrophilic reagent, to the formation of a compound of the formula $I^d$, whereafter, if desired, the compound obtained is converted to a physiologically acceptable salt thereof and/or converted to a substantially pure stereoisomer thereof.

12. A process for the preparation of a compound of the formula

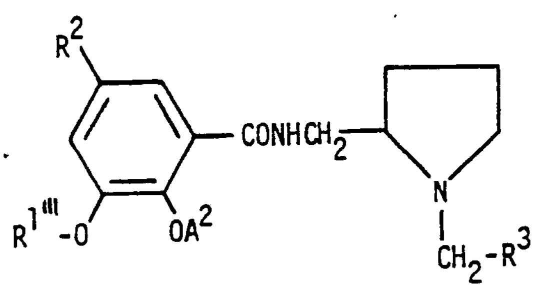

$I^{ef}$

wherein $R^2$, $R^3$ and $A^2$ have the definitions given in claim 1 and $R^{1'''}$ is an alkyl(1—4C) group, an alkyl(1—20C)-group, an alkyl(1—20C)-O—CO group, an alkyl(1—20C)-NH—CO group or an

alkyl¹ (1—20C)
\
N—CO,
/
alkyl² (1—20C)

or a physiologically acceptable salt or optical isomer thereof, which process comprises

a) O-alkylation of a compound of the formula

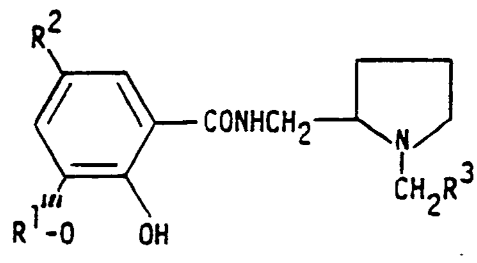

wherein $R^{1'''}$, $R^2$ and $R^3$ have the definitions given above, with a compound of the formula

$$A^2—B$$

wherein $A^2$ has the definition given above and B is a leaving group, to the formation of a compound of the formula $I^{ef}$ or

b) reaction of a compound of the formula

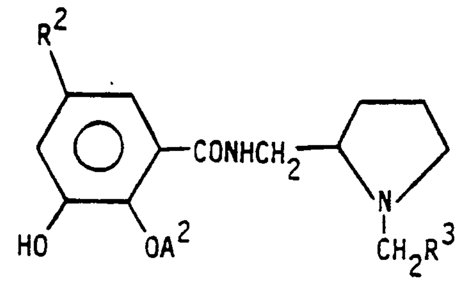

wherein $R^2$, $R^3$ and $A^2$ have the definitions given above with a compound of the formula

$$R^{1'''}—B$$

wherein $R^{1'''}$ has the definition given above and B is a leaving group to the formation of a compound of formula $I^{ef}$, whereafter, if desired, the compound obtained by any of methods e)—f) is converted to a physiologically acceptable salt thereof and/or converted to a substantially pure stereoisomer thereof.

13. A process according to any of claims 9—12 characterized in that a compound according to any of claims 1—8 is prepared.

14. A pharmaceutical preparation comprising as active ingredient a compound according to any of claims 1—8 or a physiologically acceptable salt or an optical isomer thereof.

15. A pharmaceutical preparation according to claim 14 in dosage unit form.

16. A pharmaceutical preparation according to claims 14—15 comprising the active ingredient in association with a pharmaceutically acceptable carrier.

17. The use of a compound according to any of claims 1—8 or a physiologically acceptable salt thereof for the preparation of a pharmaceutical preparation comprising as an active ingredient an amount of the said compound.

18. A compound according to any of claims 1—8 for use as a drug.

**Claims for the Contracting State: AT**

1. A process for the preparation of a compound of the formula

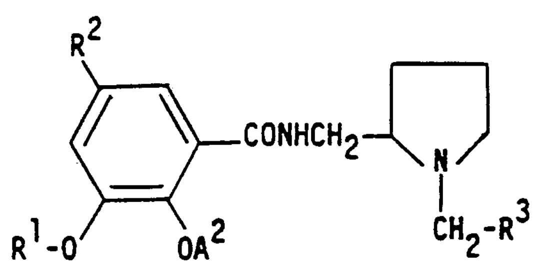

wherein

$R^1$ is a hydrogen atom, an alkyl (1—4C) group, an alkyl(1—20C)-CO group, an alkyl(1—20)-O—CO group, an alkyl(1—20)-NH—CO group or an

```
alkyl¹(1—20C)
             \
              N—CO group;
             /
alkyl²(1—20C)
```

wherein $alkyl^1$ and $alkyl^2$ can be the same or different,

$R^2$ is a halogen atom, an alkyl(1—4C) group or an alkyl(1—4C)-thio group;

$A^2$ is a methyl or ethyl group; and

$R^3$ is a hydrogen atom, an alkyl(1—4C) group, an alkenyl(2—3C) group or a phenyl group substituted by one or more of fluoro, chloro, bromo, trifluoromethyl, ethyl, methoxy or ethoxy in the orto, meta or para positions, or substituted by methylenedioxy;

or a physiologically acceptable salt or optical isomer thereof, which process comprises

a) reaction of a compound of the formula

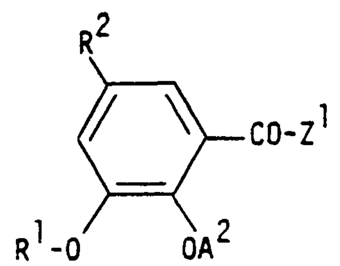

wherein $R^1$, $R^2$ and $A^2$ have the definitions given above and —CO—$Z^1$ is a reactive group capable of reacting with an amino group under formation of an amide moiety, with a compound of the formula

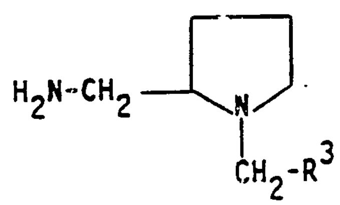

wherein $R^3$ has the definition given above, or a reactive derivative thereof, to the formation of a compound of the formula I or

b) N-substitution of a compound of the formula

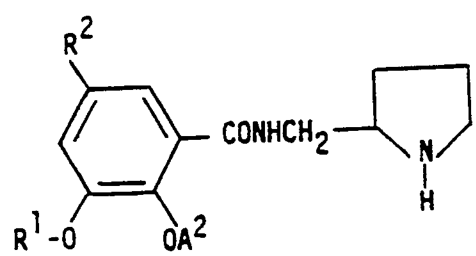

wherein $R^1$, $R^2$ and $A^2$ have the definitions given above with a compound of the formula

1) $R^3$—$CH_2$—X; or

2) $R^3CHO$ in the presence of a reducing agent; or

3) $R^3COCl$, $(R^3CO)_2O$ or $R^3$—$COOCH_3$ and subsequent reduction,

wherein $R^3$ has the definition given above and X is a leaving group, to the formation of a compound of the formula I, whereafter, if desired, the compound obtained by any of the methods a)—b) is converted to a physiologically acceptable salt thereof and/or converted to a substantially pure stereoisomer thereof.

2. A process for the preparation of a compound of the formula

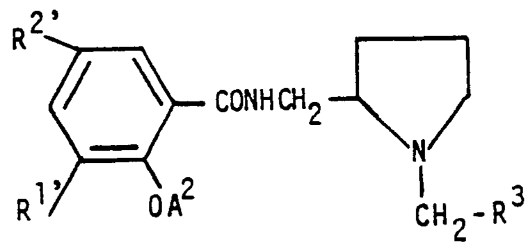

I$^{c}$

wherein $A^2$ and $R^2$ have the definitions given in claim 1 and $R^{1'}$ is a hydrogen atom or an alkyl(1—4C) group

and $R^{2'}$ is a bromine or chlorine atom, or a physiologically acceptable salt or optical isomer thereof, which process comprises reaction of a compound of the formula

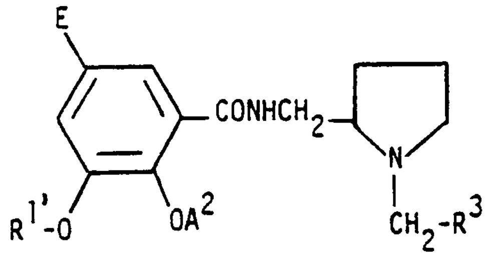

wherein $R^{1'}$, $A^2$ and $R^3$ have the definitions given above and E is hydrogen or $Si(CH_3)_3$, with a chlorinating or brominating reagent, to the formation of a compound of the formula $I^c$, whereafter, if desired, the compound obtained is converted to a physiologically acceptable salt thereof and/or converted to a substantially pure stereoisomer thereof.

3. A process for the preparation of a compound of the formula

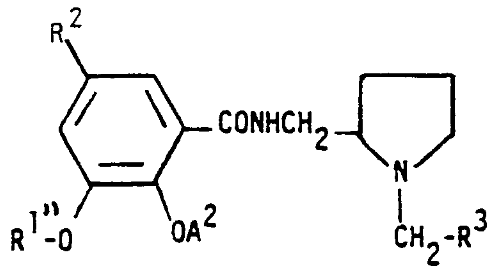

$I^d$

wherein $R^2$, $R^3$ and $A^2$ have the definitions given in claim 1 and $R^{1''}$ is an alkyl(1—4C) group, or a physiologically acceptable salt or optical isomer thereof, which process comprises reaction of a compound of the formula

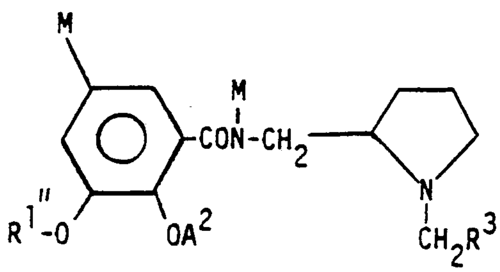

wherein $R^{1''}$, $R^3$ and $A^2$ have the definitions given above and M is a metal derivative, with an electrophilic reagent, to the formation of a compound of the formula $I^d$, whereafter, if desired, the compound obtained is converted to a physiologically acceptable salt thereof and/or converted to a substantially pure stereoisomer thereof.

4. A process for the preparation of a compound of the formula

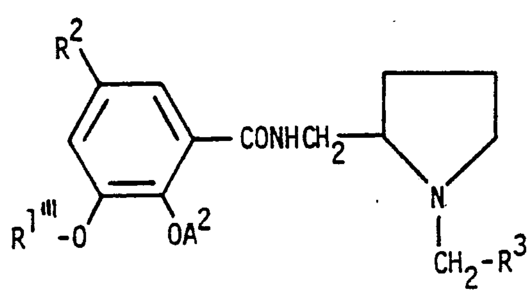

wherein $R^2$, $R^3$ and $A^2$ have the definitions given in claim 1 and $R^{1'''}$ is an alkyl(1—4C) group, an alkyl(1—20C)-group, an alkyl(1—20C)-O—CO group, an alkyl(1—20C)-NH—CO group or an

alkyl$^1$ (1—20C)
\
N—CO,
/
alkyl$^2$ (1—20C)

or a physiologically acceptable salt or optical isomer thereof, which process comprises

a) O-alkylation of a compound of the formula

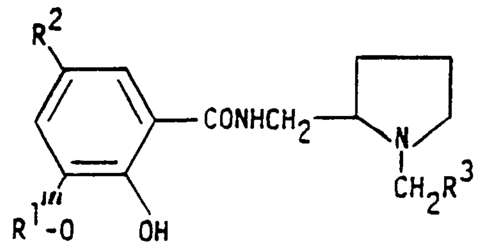

$I^{ef}$

wherein $R^{1'''}$, $R^2$ and $R^3$ have the definitions given above, with a compound of the formula

$A^2$—B

wherein $A^2$ has the definition given above and B is a leaving group, to the formation of a compound of the formula $I^{ef}$ or

b) reaction of a compound of the formula

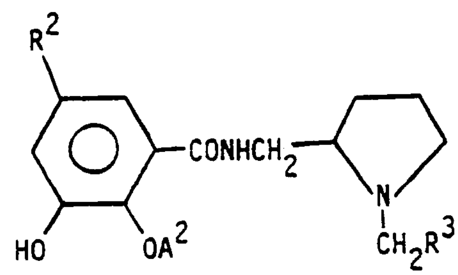

wherein $R^2$, $R^3$ and $A^2$ have the definitions given above with a compound of the formula

$R^{1'''}$—B

wherein $R^{1'''}$ has the definition given above and B is a leaving group to the formation of a compound of formula $I^{ef}$, whereafter, if desired, the compound obtained by any of methods e)—f) is converted to a physiologically acceptable salt thereof and/or converted to a substantially pure stereoisomer thereof.

5. A process according to any of claims 1—4 wherein a compound of the formula I is prepared, in which formula

$R^1$ is hydrogen, methyl, ethyl or an alkyl(1—20C)-CO group;
$R^2$ is halogen or alkyl;
$A^2$ is methyl or ethyl; and
$R^3$ has the definition given in claim 1.

6. A process according to any of claims 1—4 wherein a compound of the formula I is prepared, in which formula

$R^1$ is hydrogen, methyl or ethyl;
$R^2$ is chlorine, bromine, iodine, methyl, ethyl, n-propyl or n-butyl;
$A^2$ is methyl or ethyl; and
$R^3$ has the definition given in claim 1.

7. A process according to any of claims 1—4 wherein a compound of the formula I is prepared, in which formula

$R^1$ is methyl;
$R^2$ is chlorine, bromine, iodine, methyl, ethyl, n-propyl or n-butyl;
$A^2$ is methyl; and
$R^3$ is a hydrogen atom, an alkyl(1—4C) group, an alkenyl(2—3C) group, or a phenyl group substituted in the para position with a fluorine atom.

8. A process according to any of claims 1—4 wherein a compound of the formula I is prepared, in which formula

$R^1$ is methyl;
$R^2$ is bromine;
$A^2$ is methyl; and
$R^3$ has the definition given in claim 7.

9. A process according to any of claims 1—4 wherein a compound of the formula

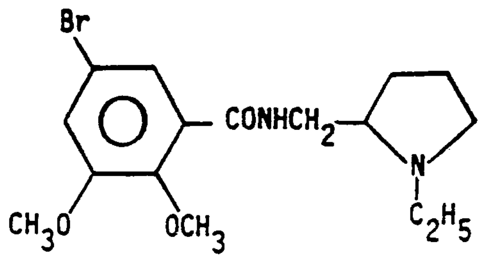

or

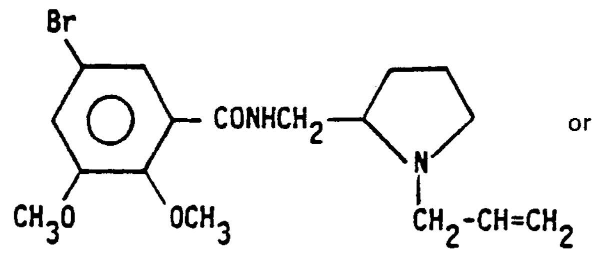

or

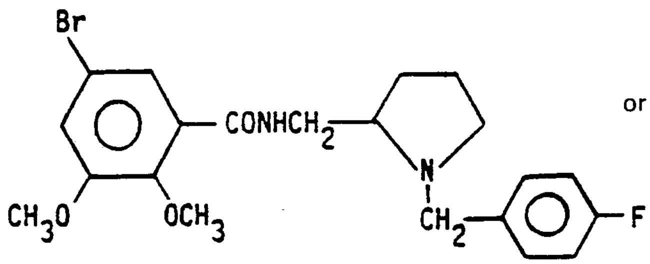

or

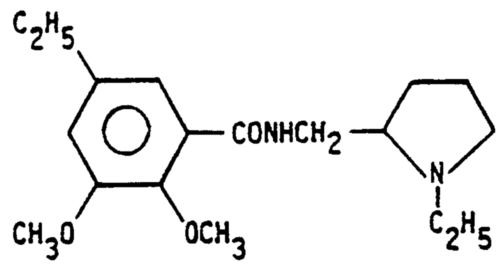

or

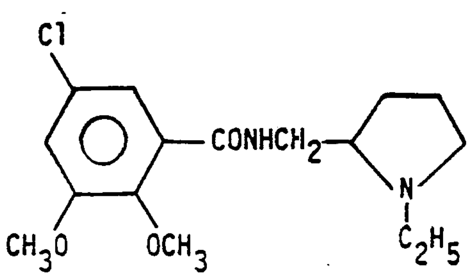

or

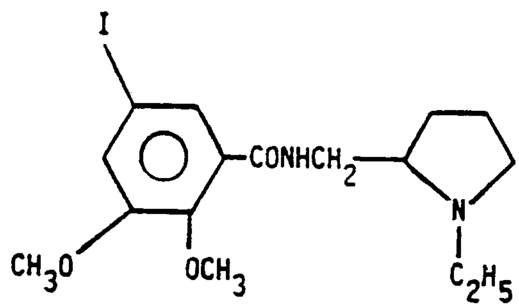

or

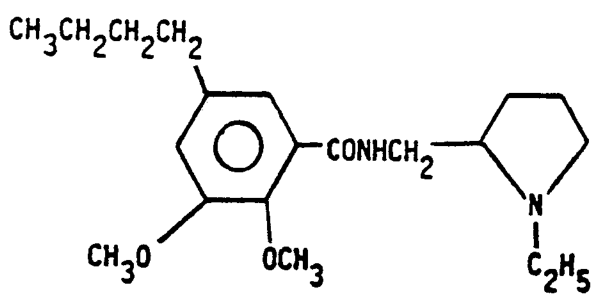

is prepared.

10. A process according to any of claims 1—9 wherein the compound is prepared in the form of an optical isomer.

11. A process according to any of claims 1—10 wherein the compound is prepared in the form of a physiologically acceptable salt.

**Patentansprüche für die Vertragsstaaten: BE CH DE FR GB IT LI LU NL SE**

1. Verbindung der Formel

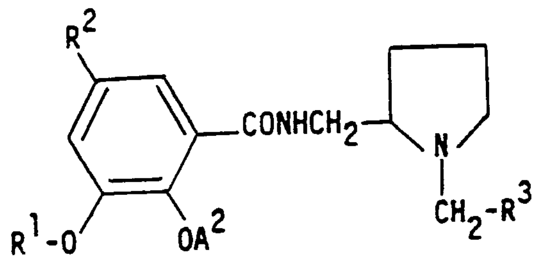

worin

$R^1$ ist: ein Wasserstoffatom, eine Alkyl(1—4C)-Gruppe, eine Alkyl(1—20C)—CO-gruppe, eine Alkyl(1—20)—O—CO-Gruppe, eine Alkyl(1—20)—NH—CO-Gruppe oder eine

Alkyl¹(1—20C)
\
N—CO-Gruppe,
/
Alkyl²(1—20C)

worin Alkyl¹ und Alkyl² gleich oder verschieden sein können;

$R^2$ ist: ein Halogenatom, eine Alkyl(1—4C)-Gruppe oder eine Alkyl(1—4C)-thiogruppe;

$A^2$ ist: eine Methyl- oder Ethylgruppe; und

$R^3$ ist: ein Wasserstoffatom, eine Alkyl(1—4C)-Gruppe, eine Alkenyl(2—3C)-Gruppe oder eine Phenylgruppe substituiert durch eines oder mehrere von Fluor, Chlor, Brom, Trifluormethyl, Ethyl, Methoxy oder Ethoxy an den Ortho-, Meta- oder Para-Positionen oder substituiert durch Methylendioxy; oder ein physiologisch akzeptables Salz oder optisches Isomeres davon.

2. Verbindung nach Anspruch 1 mit der Formel I, worin

$R^1$ ist: Wasserstoff, Methyl, Ethyl oder eine Alkyl(1—20C)—CO-Gruppe;

$R^2$ ist: Halogen oder Alkyl;

$A^2$ ist: Methyl oder Ethyl; und

$R^3$ die im Anspruch 1 angegebene Bedeutung hat.

3. Verbindung nach Anspruch 1 mit der Formel I, worin

$R^1$ ist: Wasserstoff, Methyl oder Ethyl;

$R^2$ ist: Chlor, Brom, Jod, Methyl, Ethyl, n-Propyl oder n-Butyl;

$A^2$ ist: Methyl oder Ethyl; und

$R^3$ die im Anspruch 1 angegebene Bedeutung hat.

4. Verbindung nach Anspruch 1 mit der Formel I, worin

$R^1$ Methyl ist;

$R^2$ ist: Chlor, Brom, Jod, Methyl, Ethyl, n-Propyl oder n-Butyl;

$A^2$ Methyl ist; und

$R^3$ ist: ein Wasserstoffatom, eine Alkyl(1—4C)-Gruppe, eine Alkenyl(2—3C)-Gruppe oder eine Phenylgruppe substituiert mit einem Fluoratom an der Para-Position.

5. Verbindung nach Anspruch 1 mit der Formel I, worin

$R^1$ Methyl ist;

$R^2$ Brom ist;

$A^2$ Methyl ist;

$R^3$ die im Anspruch 4 angegebene Bedeutung hat.

6. Verbindung nach Anspruch 1 mit der Formel

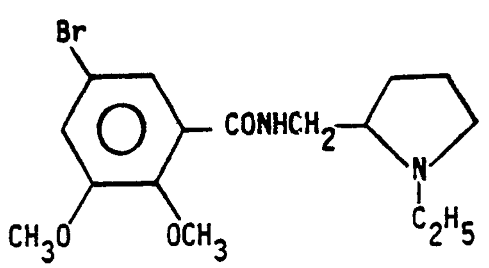 ; 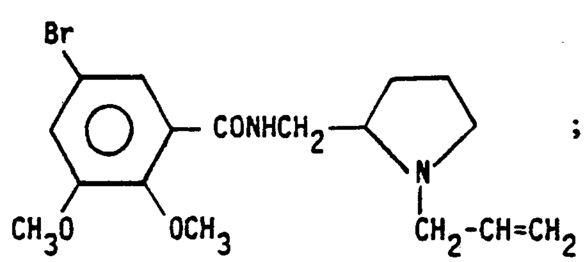 ;

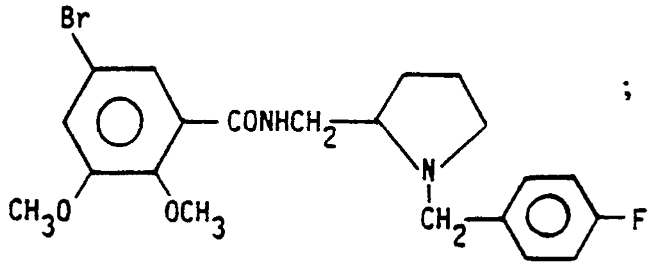 ; 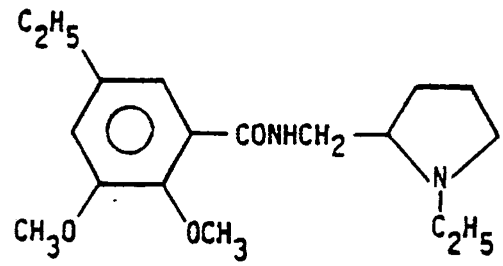 ;

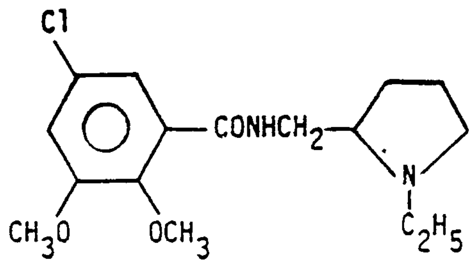 ; 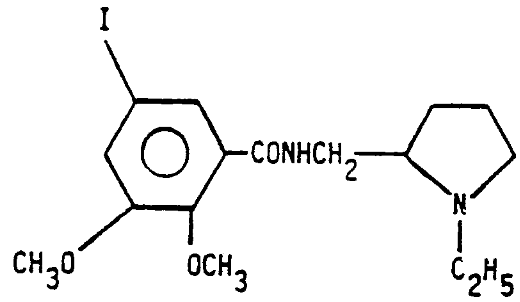 ;

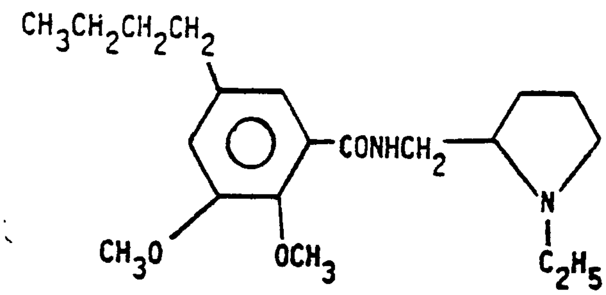

7. Verbindung nach irgendeinem der Ansprüche 1 bis 6 in Form eines optischen Isomeren davon.

8. Verbindung nach irgendeinem der Ansprüche 1 bis 7 in Form eines physiologisch akzeptablen Salzes davon.

9. Verfahren zur Herstellung einer Verbindung der Formel

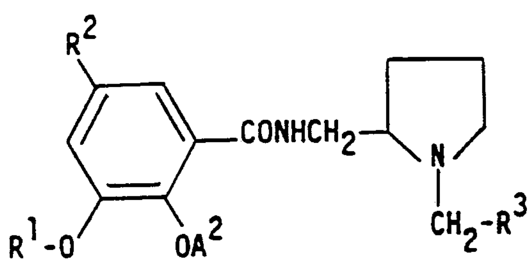

I

worin $R^1$, $R^2$, $R^3$ und $A^2$ die im Anspruch 1 angegebenen Bedeutungen haben, oder eines physiologisch akzeptablen Salzes oder optischen Isomeren davon, welches Verfahren umfaßt:

a) die Umsetzung einer Verbindung der Formel

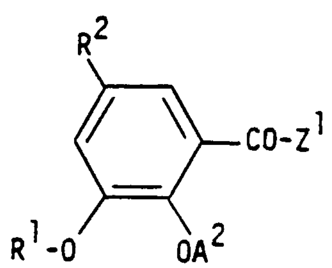

worin $R^1$, $R^2$ und $A^2$ die oben angeführten Bedeutungen haben, und —CO—$Z^1$ eine reaktive Gruppe ist,

welche fähig ist, mit einer Aminogruppe unter Bildung eines Amidanteils zu reagieren, mit einer Verbindung der Formel

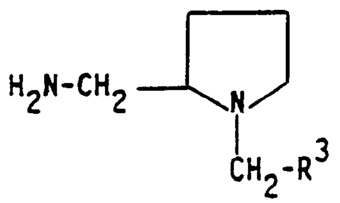

worin $R^3$ die oben angegebene Bedeutung hat, oder einem reaktiven Derivat davon zur Bildung einer Verbindung der Formel I oder

b) die N-Substitution einer Verbindung der Formel

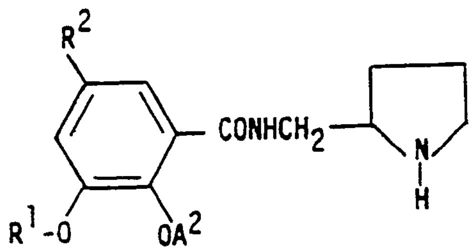

worin $R^1$, $R^2$ und $A^2$ die oben angegebenen Bedeutungen haben, mit einer Verbindung der Formel

1) $R^3—CH_2—X$; oder

2) $R^3CHO$ in Anwesenheit eines Reduktoinsmittels; oder

3) $R^3COCl$, $(R^3CO)_2O$ oder $R^3—COOCH_3$ und darauffolgende Reduktion, worin $R^3$ die oben angegebene Bedeutung hat, und X eine austretende Gruppe ist, zur Bildung einer Verbindung der Formel I, wonach gewünschtenfalls die gemäß irgendeinem der Verfahren a)—b) erhaltene Verbindung in ein physiologisch akzeptables Salz davon übergeführt wird und/oder in ein im wesentlichen reines Stereoisomeres davon übergeführt wird.

10. Verfahren zur Herstellung einer Verbindung der Formel

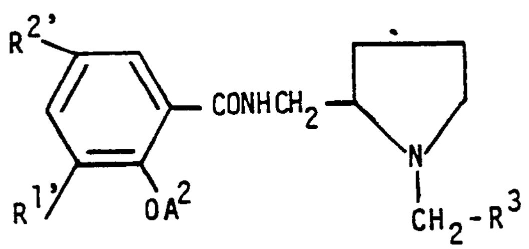

$I^c$

worin $A^2$ und $R^2$ die im Anspruch 1 angegebenen Bedeutungen haben, und $R^{1'}$ ein Wasserstoffatom oder eine Alkyl(1—4C)-Gruppe ist, und $R^{2'}$ ein Brom- oder Chloratom ist, oder eines physiologisch akzeptablen Salzes oder optischen Isomeren davon, welches Verfahren umfaßt:
die Umsetzung einer Verbindung der Formel

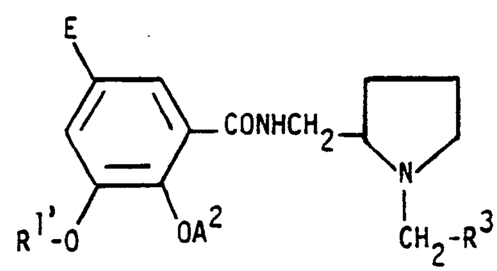

worin $R^{1'}$, $A^2$ und $R^3$ die oben angegebenen Bedeutungen haben, und E Wasserstoff oder $Si(CH_3)_3$ ist, mit einem Chlorierungs- oder Bromierungsreagens zur Bildung einer Verbindung der Formel $I^c$, wonach gewünschtenfalls die erhaltene Verbindung in ein physiologisch akzeptables Salz davon übergeführt wird

und/oder in ein im wesentlichen reines Stereoisomeres davon übergeführt wird.

11. Verfahren zur Herstellung einer Verbindung der Formel

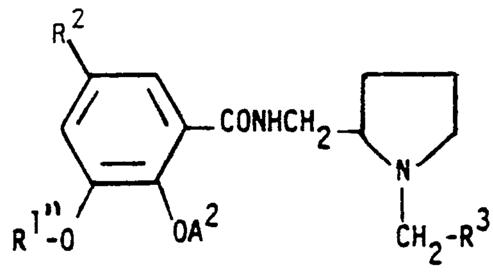

$I^d$

worin $R^2$, $R^3$ und $A^2$ die im Anspruch 1 angegebenen Bedeutungen haben, und $R^{1''}$ eine Alkyl(1—4C)-Gruppe ist, oder eines physiologisch akzeptablen Salzes oder optischen Isomeren davon, welches Verfahren umfaßt;
die Umsetzung einer Verbindung der Formel

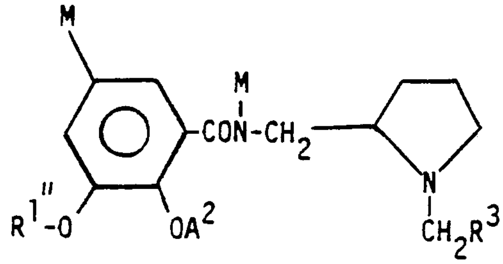

worin $R^{1''}$, $R^3$ und $A^2$ die oben angegebenen Bedeutungen haben, und M ein Metallderivat ist, mit einem elektrophilen Reagens zur Bildung einer Verbindung der Formel $I^d$, wonach gewünschtenfalls die erhaltene Verbindung in ein physiologisch akzeptables Salz davon übergeführt wird und/oder in ein im wesentlichen reines Stereoisomeres davon übergeführt wird.

12. Verfahren zur Herstellung einer Verbindung der Formel

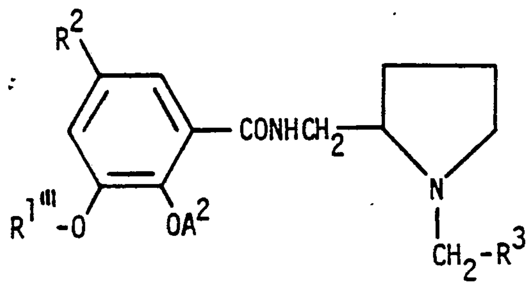

$I^{ef}$

worin $R^2$, $R^3$ und $A^2$ die im Anspruch 1 angegebenen Bedeutungen haben, und $R^{1'''}$ ist: eine Alkyl(1—4C)-Gruppe, eine Alkyl(1—20C)—CO-Gruppe, eine Alkyl(1—20C)—O—CO-Gruppe, eine Alkyl(1—20C)—NH—CO-Gruppe oder ein

Alkyl$^1$(1—20C)
\
N—CO,
/
Alkyl$^2$(1—20C)

oder eines physiologisch akzeptablen Salzes oder optischen Isomeren davon, welches Verfahren umfaßt:

a) O-Alkylierung einer Verbindung der Formel

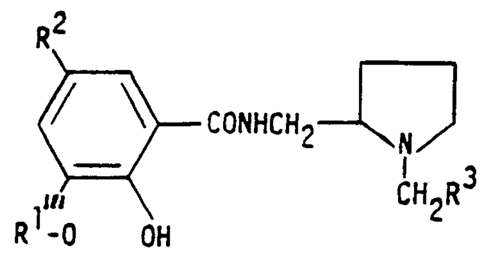

worin $R^{1'''}$, $R^2$ und $R^3$ die oben angegebenen Bedeutungen haben, mit einer Verbindung der Formel $A^2$—B, worin $A^2$ die oben angegebene Bedeutung hat, und B eine austretende Gruppe ist, zur Bildung einer Verbindung der Formel $I^{ef}$ oder

b) die Umsetzung einer Verbindung der Formel

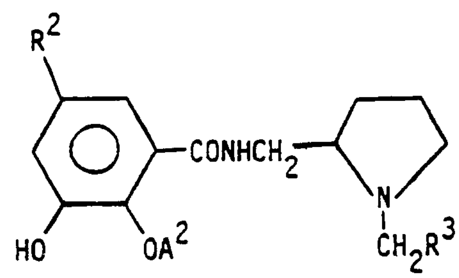

worin $R^2$, $R^3$ und $A^2$ die oben angegebenen Bedeutungen haben, mit einer Verbindung der Formel $R^{1'''}$—B, worin $R^{1'''}$ die oben angegebene Bedeutung hat, und B eine austretende Gruppe ist, zur Bildung einer Verbindung der Formel $I^{ef}$, wonach gewünschtenfalls die gemäß irgendeinem der Verfahren e)—f) erhaltene Verbindung in ein physiologisch akzeptables Salz davon übergeführt wird und/oder in ein im wesentlichen reines Stereoisomeres davon übergeführt wird.

13. Verfahren nach irgendeinem der Ansprüche 9 bis 12, dadurch gekennzeichnet, daß eine Verbindung nach irgendeinem der Ansprüche 1 bis 8 hergestellt wird.

14. Pharmazeutische Präparation, welche als aktives Ingrediens eine Verbindung nach irgendeinem der Ansprüche 1 bis 8 oder ein physiologisch akzeptables Salz oder ein optisches Isomeres davon umfaßt.

15. Pharmazeutische Präparation nach Anspruch 14 in Dosiseinheitsform.

16. Pharmazeutische Präparation nach den Ansprüchen 14 bis 15, welche das aktive Ingrediens in Verbindung mit einem pharmazeutisch akzeptablen Träger umfaßt.

17. Verbindung nach irgendeinem de Ansprüche 1 bis 8 oder ein physiologisch akzeptables Salz davon zur Verwendung bei der Herstellung einer pharmazeutischen Präparation, welche als aktives Ingrediens eine Menge der Verbindung enthält.

18. Verbindung nach irgendeinem der Ansprüche 1 bis 8 zur Verwendung als Arzneimittel.

**Patentansprüche für den Vertragsstaat: AT**

1. Verfahren zur Herstellung einer Verbindung der Formel

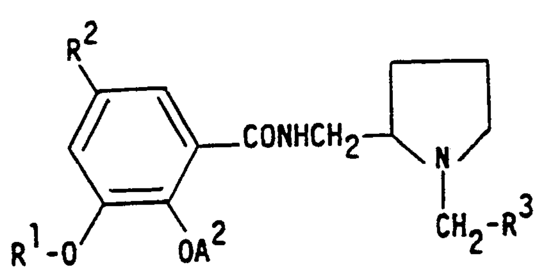

worin

$R^1$ ist: ein Wasserstoffatom, eine Alkyl(1—4C)-Gruppe, eine Alkyl(1—20C)—CO-Gruppe, eine Alkyl(1—20)—O—CO-Gruppe, eine Alkyl(1—20)—NH—CO-Gruppe oder eine

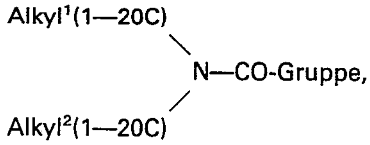

worin Alkyl[1] und Alkyl[2] gleich oder verschieden sein können;

$R^2$ ist: ein Halogenatom, eine Alkyl(1—4C)-Gruppe oder eine Alkyl(1—4C)-thiogruppe;

$A^2$ ist: eine methyl- oder Ethylgruppe; und

$R^3$ ist: ein Wasserstoffatom, eine Alkyl(1—4C)-Gruppe, eine Alkenyl(2—3C)-Gruppe oder eine Phenylgruppe substituiert durch eines oder mehrere von Fluor, Chlor, Brom, Trifluormethyl, Ethyl, Methoxy oder Ethoxy an den Ortho-, Meta- oder Para-Positionen oder substituiert durch Methylendioxy; oder eines physiologisch akzeptablen Salzes oder optischen Isomeren davon, welches Verfahren umfaßt:

a) die Umsetzung einer Verbindung der Formel

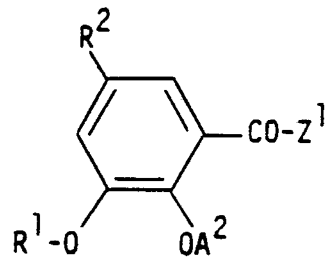

worin $R^1$, $R^2$ und $A^2$ die oben angegebenen Bedeutungen haben, und —CO—$Z^1$ eine reaktive Gruppe ist, welche fähig ist, mit einer Aminogruppe unter Bildung eines Amidanteils zu reagieren, mit einer Verbindung der Formel

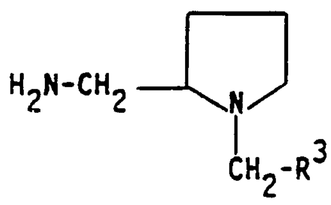

worin $R^3$ die oben angegebene Bedeutung hat, oder einem reaktiven Derivat davon zur Bildung einer Verbindung der Formel I oder

b) die N-Substitution einer Verbindung der Formel

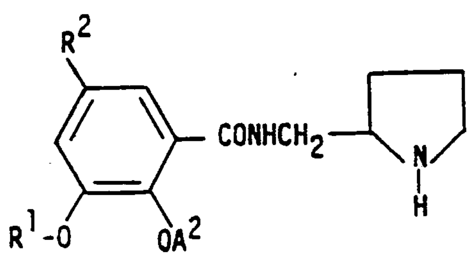

worin $R^1$, $R^2$ und $A^2$ die oben angegebenen Bedeutungen haben, mit einer Verbindung der Formel

1) $R^3$—$CH_2$—X; oder

2) $R^3CHO$ in Anwesenheit eines Reduktoinsmittels; oder

3) $R^3COCl$, $(R^3CO)_2O$ oder $R^3$—$COOCH_3$ und darauffolgende Reduktion, worin $R^3$ die oben angegebene Bedeutung hat, und X eine austretende Gruppe ist, zur Bildung einer Verbindung der Formel I, wonach gewünschtenfalls die gemäß irgendeinem der Verfahren a)—b) erhaltene Verbindung in ein physiologisch akzeptables Salz davon übergeführt wird und/oder in ein im wesentlichen reines Stereoisomeres davon übergeführt wird.

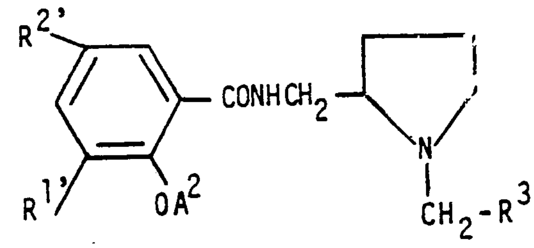

$I^c$.

worin $A^2$ und $R^2$ die im Anspruch 1 angegebenen Bedeutungen haben, und $R^{1'}$ ein Wasserstoffatom oder eine Alkyl(1—4C)-Gruppe ist, und $R^{2'}$ ein Brom- oder Chloratom ist, oder eines physiologisch akzeptablen Salzes oder optischen Isomeren davon, welches Verfahren umfaßt:
die Umsetzung einer Verbindung der Formel

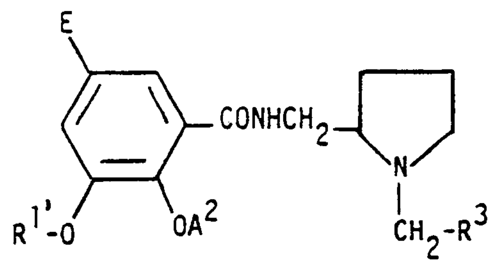

worin $R^{1'}$, $A^2$ und $R^3$ die oben angegebenen Bedeutungen haben, und E Wasserstoff oder $Si(CH_3)_3$ ist, mit einem Chlorierungs- oder Bromierungsreagens zur Bildung einer Verbindung der Formel $I^c$, wonach gewünschtenfalls die erhaltene Verbindung in ein physiologisch akzeptables Salz davon übergeführt wird und/oder in ein im wesentlichen reines Stereoisomeres davon übereführt wird.

3. Verfahren zur Herstellung einer Verbindung der Formel

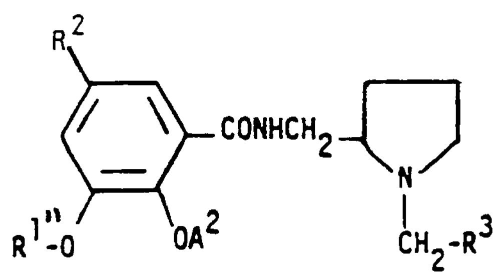

$I^d$

worin $R^2$, $R^3$ und $A^2$ die im Anspruch 1 angegebenen Bedeutungen haben, und $R^{1''}$ eine Alkyl(1—4C)-Gruppe ist, oder eines physiologisch akzeptablen Salzes oder optischen Isomeren davon, welches Verfahren umfaßt;
die Umsetzung einer Verbindung der Formel

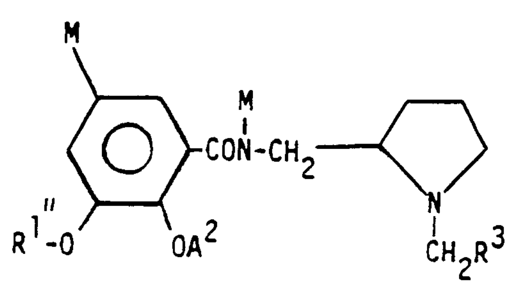

worin $R^{1''}$, $R^3$ und $A^2$ die oben angegebenen Bedeutungen haben, und M ein Metallderivat ist, mit einem elektrophilen Reagens zur Bildung einer Verbindung der Formel $I^d$, wonach gewünschtenfalls die erhaltene Verbindung in ein physiologisch akzeptables Salz davon übergeführt wird und/oder in ein im wesentlichen reines Stereoisomeres davon übergeführt wird.

4. Verfahren zur Herstellung einer Verbindung der Formel

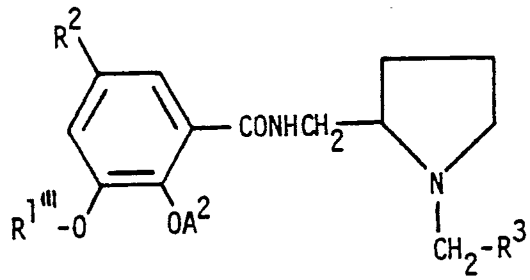

worin $R^2$, $R^3$ und $A^2$ die im Anspruch 1 angegebenen Bedeutungen haben, und $R^{1'''}$ ist: eine Alkyl(1—4C)-Gruppe, eine Alkyl(1—20C)—CO-Gruppe, eine Alkyl(1—20C)—O—CO-Gruppe, eine Alkyl(1—20C)—NH—CO-Gruppe oder ein

Alkyl$^1$(1—20C)
\
N—CO,
/
Alkyl$^2$(1—20C)

oder eines physiologisch akzeptablen Salzes oder optischen Isomeren davon, welches Verfahren umfaßt:

a) die O-Alkylierung einer Verbindung der Formel

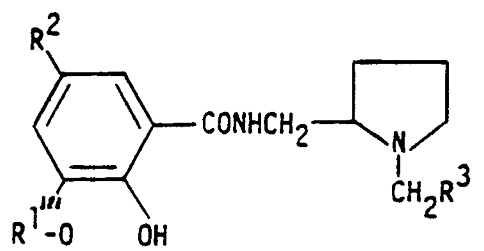

$I^{ef}$

worin $R^{1'''}$, $R^2$ und $R^3$ die oben angegebenen Bedeutungen haben, mit einer Verbindung der Formel $A^2$—B, worin $A^2$ die oben angegebene Bedeutung hat, und B eine austretende Gruppe ist, zur Bildung einer Verbindung der Formel $I^{ef}$ oder

b) die Umsetzung einer Verbindung der Formel

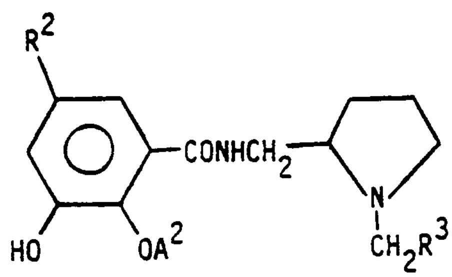

worin $R^2$, $R^3$ und $A^2$ die oben angegebenen Bedeutungen haben, mit einer Verbindung der Formel $R^{1'''}$—B, worin $R^{1'''}$ die oben angegebene Bedeutung hat, und B eine austretende Gruppe ist, zur Bildung einer Verbindung der Formel $I^{ef}$, wonach gewünschtenfalls die gemäß irgendeinem der Verfahren e)—f) erhaltene Verbindung in ein physiologisch akzeptables Salz davon übergeführt wird und/oder in ein im wesentlichen reines Stereoisomeres davon übergeführt wird.

5. Verfahren nach irgendeinem der Ansprüche 1 bis 4, worin eine Verbindung der Formel I hergestellt wird, in welche Formel

$R^1$ ist: Wasserstoff, Methyl, Ethyl oder eine Alkyl(1—20C)—CO-Gruppe;
$R^2$ Halogen oder Alkyl ist;
$A^2$ Methyl oder Ethyl ist; und
$R^3$ die im Anspruch 1 angegebene Bedeutung hat.

6. Verfahren nach irgendeinem der Ansprüche 1 bis 4, worin eine Verbindung der Formel I hergestellt wird, in welcher Formel

$R^1$ ist: Wasserstoff, Methyl oder Ethyl;

$R^2$ ist: Chlor, Brom, Jod, Methyl, Ethyl, n-Propyl oder n-Butyl;

$A^2$ Methyl oder Ethyl ist; und

$R^3$ die im Anspruch 1 angegebene Bedeutung hat.

7. Verfahren nach irgendeinem der Ansprüche 1—4, worin eine Verbindung der Formel I hergestellt wird, in welcher Formel

$R^1$ Methyl ist;

$R^2$ ist: Chlor, Brom, Jod, Methyl, Ethyl, n-Propyl oder n-Butyl;

$A^2$ Methyl ist; und

$R^3$ ist: ein Wasserstoffatom, eine Alkyl(1—4C)-Gruppe, eine Alkenyl(2—3C)-Gruppe oder eine an der Para-Position mit einem Fluoroatom substituierte Phenylgruppe.

8. Verfahren nach irgendeinem der Ansprüche 1 bis 4, worin eine Verbindung der Formel I hergestellt wird, in welcher Formel

$R^1$ Methyl ist;

$R^2$ Brom ist;

$A^3$ Methyl ist; und

$R^3$ die im Anspruch 7 angegebene Bedeutung hat.

9. Verfahren nach irgendeinem der Ansprüche 1 bis 4, worin eine Verbindung der Formel

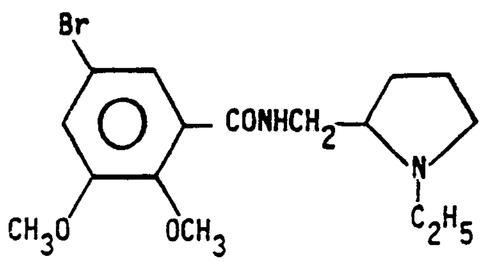

oder

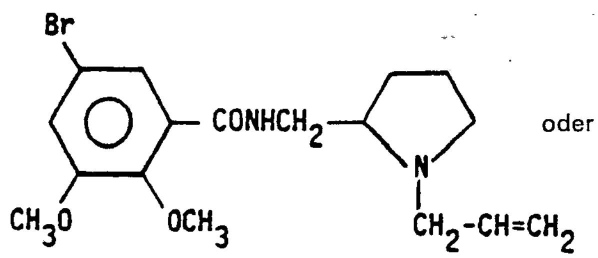

oder

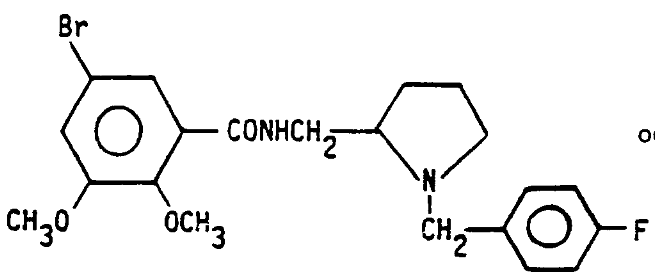

oder

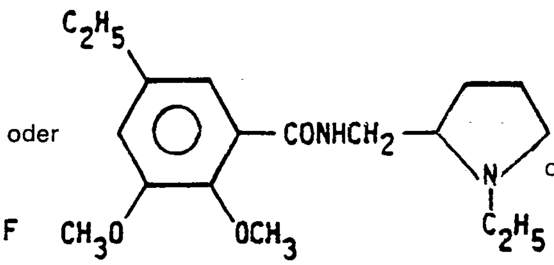

oder

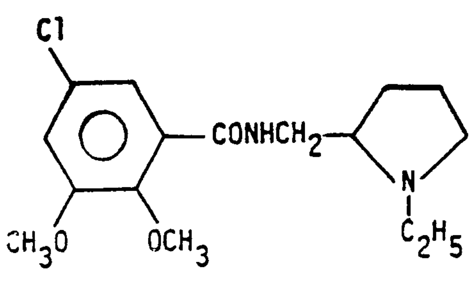

oder

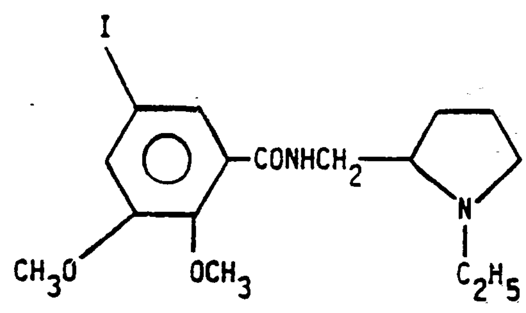

oder

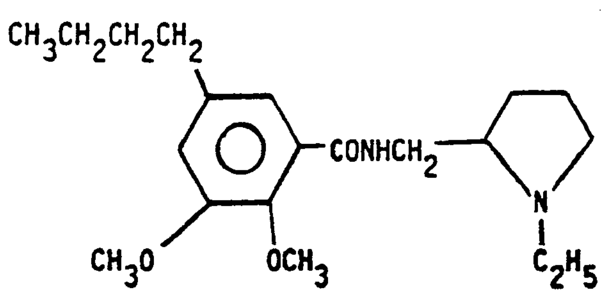

hergestellt wird.

10. Verfahren nach irgendeinem der Ansprüche 1 bis 9, worin die Verbindung in Form eines optischen Isomeren hergestellt wird.

11. Verfahren nach irgendeinem der Ansprüche 1 bis 10, worin die Verbindung in Form eines physiologisch akzeptablen Salzes hergestellt wird.

**Revendications pour les Etats contractants: BE CH DE FR GB IT LI LU NL SE**

1. Composé de formule:

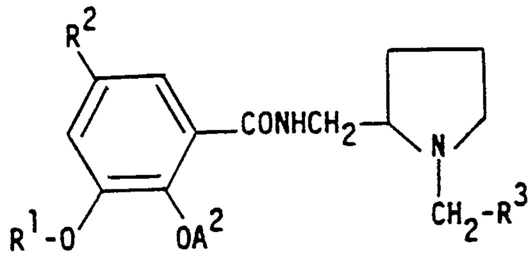

dans laquelle:

$R^1$ représente un atome d'hydrogène, un groupe alkyle ayant 1 à 4 atomes de carbone, un groupe alkyl (en $C_1$—$C_{20}$)-CO, un groupe alkyl (1 à 20 atomes de carbone)—O—CO, un groupe alkyl (1 à 20)—NH—CO ou un groupe

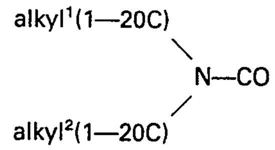

dans lequel les groupes alkyle$^1$ et alkyle$^2$ peuvent être identiques ou différents,

$R^2$ représente un atome d'halogène, un groupe alkyle (ayant 1 à 4 atomes de carbone) ou un groupe alkyl (1 à 4 atomes de carbone)-thio,

$A^2$ représente un groupe méthyle ou éthyle, et

$R^3$ représente un atome d'hydrogène, un groupe alkyle (ayant 1 à 4 atomes de carbone), un groupe alcényle (ayant 2 ou 3 atomes de carbone), ou un groupe phényle, substitué par un ou plusieurs groupes fluoro, chloro, bromo, trifluorométhyle, éthyle, méthoxy ou éthoxy en position(s) ortho, méta ou para, ou substitué par un groupe méthylènedioxy;

ou un sel physiologiquement acceptable ou un isomère actif de celui-ci.

2. Composé selon la revendication 1 répondant à la formule I, dans laquelle:

$R^1$ représente un atome d'hydrogène, un groupe méthyle, éthyle ou un groupe alkyl (1 à 20 atomes de carbone)-CO;

$R^2$ représente un atome d'halogène ou un groupe alkyle;

$A^2$ représente un groupe méthyle ou éthyle; et

$R^3$ a la définition indiquée à la revendication 1.

3. Composé selon la revendication 1 répondant à la formule I, dans laquelle:

$R^1$ représente un atome d'hydrogène, un groupe méthyle ou éthyle;

$R^2$ représente un atome de chlore, de brome, d'iode, un groupe méthyle, éthyle, n-propyle ou n-butyle;

$A^2$ représente un groupe méthyle ou éthyle; et

$R^3$ a la définition indiquée à la revendication 1.

4. Composé selon la revendication 1 répondant à la formule I, dans laquelle:

$R^1$ représente un groupe méthyle;

$R^2$ représente un atome de chlore, de brome, d'iode, un groupe méthyle, éthyle, n-propyle ou n-butyle;

$A^2$ représente un groupe méthyle; et

$R^3$ représente un atome d'hydrogène, un groupe alkyle (ayant 1 à 4 atomes de carbone), un groupe alcényle (ayant 2 ou 3 atomes de carbone), ou un groupe phényle portant comme substituant en position para un atome de fluor.

5. Composé selon la revendication 1 répondant à la formule I, dans laquelle:

$R^1$ représente un groupe méthyle;

$R^2$ représente un atome de brome;

$A^2$ représente un groupe méthyle; et

$R^3$ a la définition indiquée à la revendication 4.

6. Composé selon la revendication 1, répondant à la formule:

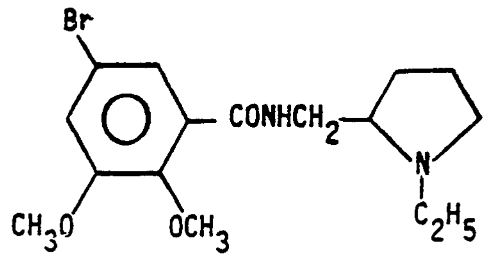 ; 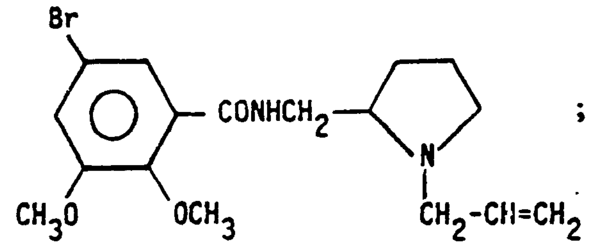 ;

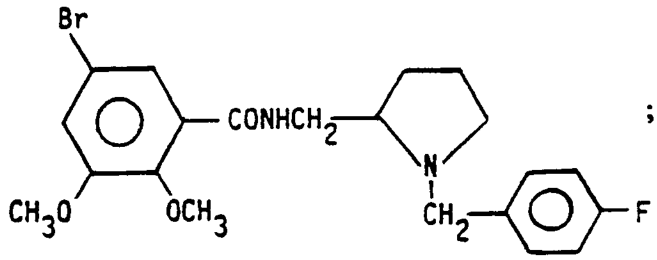 ; 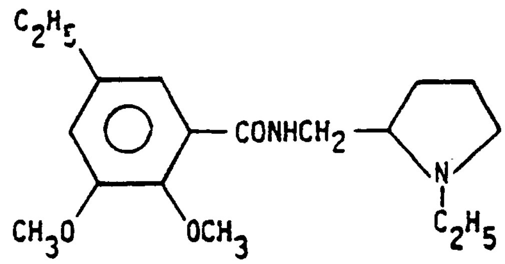 ;

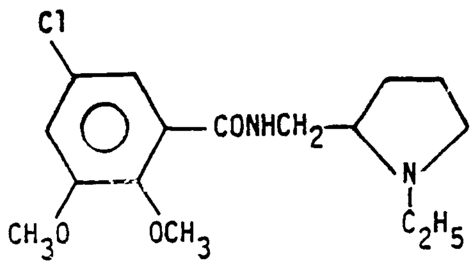 ; 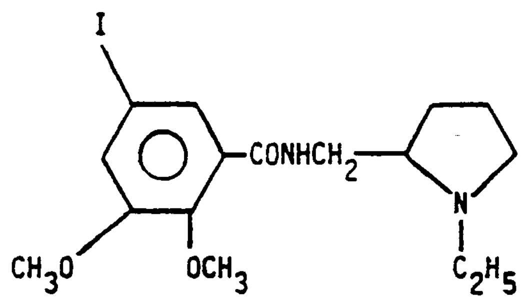 ;

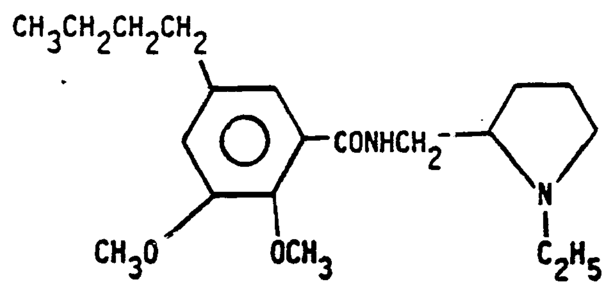

7. Composé selon l'une quelconque des revendications 1 à 6, sous forme d'un de ses isomères optiques.

8. Composé selon l'une quelconque des revendications 1 à 7, sous forme d'un sel physiologiquement acceptable.

9. Procédé pour la préparation d'un composé de formule:

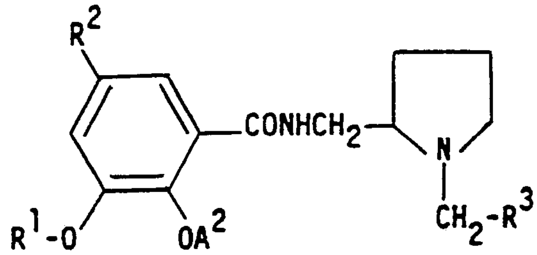 I

(dans laquelle $R^1$, $R^2$, $R^3$ et $A^2$ ont les définitions indiquées à la revendication 1) ou d'un sel

physiologiquement acceptable ou d'un isomère optique de ce composé, ce procédé comprenant:

a) la réaction d'un composé de formule:

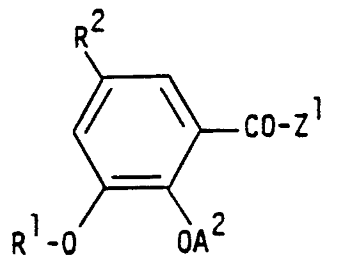

(dans laquelle $R^1$, $R^2$ et $A^2$ ont les définitions données ci-dessus, et —CO—$Z^1$ est un groupe réactif, capable de réagir avec un groupe amino, avec formation d'un fragment amide) avec un composé de formule:

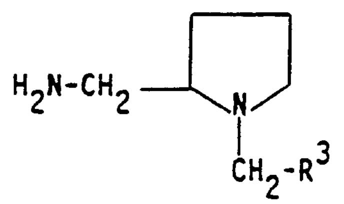

(dans laquelle $R^3$ a la définition donné ci-dessus), ou un dérivé réactif de ce composé, pour former un composé de formule I, ou

b) la réaction de substitution sur l'azote d'un composé de formule:

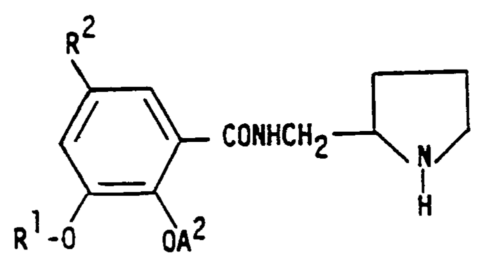

(dans laquelle $R^1$, $R^2$ et $A^2$ ont les définitions données ci-dessus) avec un composé de formule:

1) $R^3$—$CH_2$—X; ou

2) $R^3CHO$, en présence d'un agent réducteur; ou

3) $R^3COCl$, $(R^3CO)_2O$ ou $R^3$—$COOCH_3$, et une réduction subséquente,

formules dans lesquelles $R^3$ a la définition donnée ci-dessus, et X est un groupe partant, pour former un composé de formule I puis, si on le désire, on transforme le composé, obtenu selon l'une quelconque des méthodes a)—b), en un de ses sels physiologiquement acceptables et/ou on le convertit en un stéréoisomère sensiblement pur.

10. Procédé pour préparer un composé de formule:

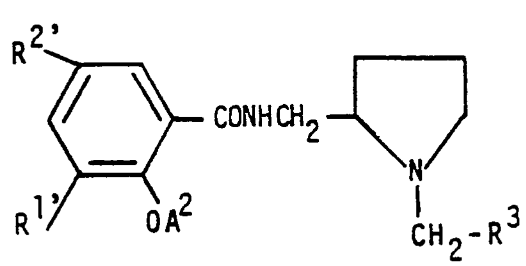

$I^c$

(dans laquelle $A^2$ et $R^2$ ont les définitions données à la revendication 1, et $R^{1'}$ représente un atome d'hydrogène ou un groupe alkyle ayant 1 à 4 atomes de carbone, et $R^{2'}$ représente un atome de brome ou de chlore) ou d'un sel physiologiquement acceptable ou d'un isomère optique de ce composé, ce procédé comprenant la réaction d'un composé de formule:

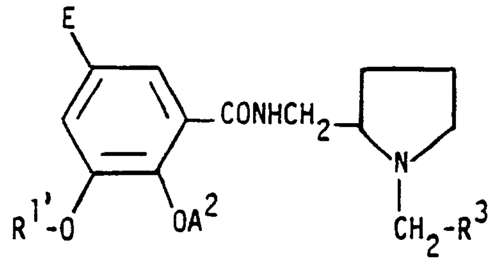

(dans laquelle $R^{1'}$, $A^2$ et $R^3$ ont les définitions données ci-dessus, et E représente un atome d'hydrogène ou $Si(CH_3)_3$), avec un réactif de chloration ou de bromation, pour former un composé de formule $I^c$, puis, si on le désire, on convertit le composé ainsi obtenu en un de ses sels physiologiquement aceptables et/ou on le convertit en un de ses stéréoisomères essentiellement purs.

11. Procédé pour préparer un composé de formule:

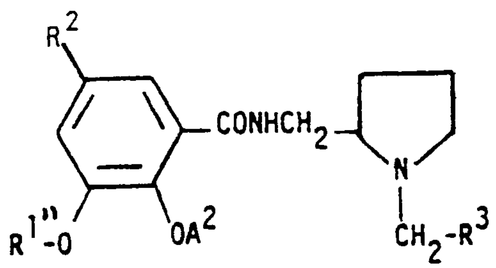

$I^d$

(dans laquelle $R^2$, $R^3$ et $A^2$ ont les définitions données à la revendication 1, et $R^{1''}$ représente un groupe alkyle ayant 1 à 4 atomes de carbone), ou d'un de ses sels physiologiquement acceptables ou d'un isomère optique, ce procédé comprenant la réaction d'un composé de formule:

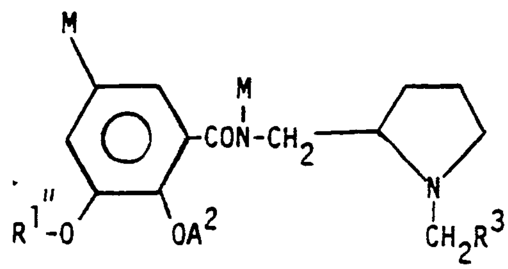

(dans laquelle $R^{1''}$, $R^3$ et $A^2$ ont les définitions indiquées ci-dessus, et M représente un dérivé de métal) avec un réactif électrophile, pour former un composé de formule $I^d$ puis, si on le désire, on convertit le composé ainsi obtenu en un de ses sels physiologiquement acceptables et/ou on le convertit en un de ses stéréoisomères essentiellement purs.

12. Procédé pour préparer un composé de formule:

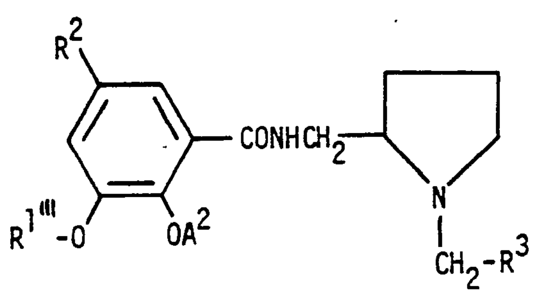

$I^{ef}$

(dans laquelle $R^2$, $R^3$ et $A^2$ ont les définitions données à la revendication 1, et $R^{1'''}$ représente un groupe alkyle (ayant 1 à 4 atomes de carbone), un groupe alkyl (ayant 1 à 20 atomes de carbone)-CO, un groupe alkyl (1 à 20 C)—O—CO, un groupe alkyl (1—20 C)—NH—CO ou un groupe:

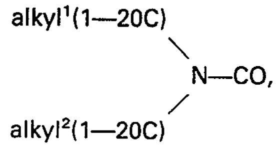

ou un de ses sels physiologiquement acceptables ou un isomere optique de ce composé, ce procédé comprenant:

a) une O-alkylation d'un composé de formule:

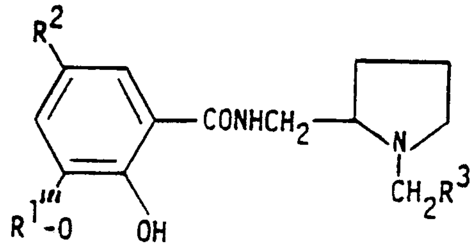

(dans laquelle $R^{1'''}$, $R^2$ et $R^3$ ont les définitions données ci-dessus) avec un composé de formule:

$$A^2—B$$

(dans laquelle $A^2$ a la définition donnée ci-dessus et B représente un groupe partant), pour former un composé de formule $I^{ef}$, ou

b) la réaction d'un composé de formule:

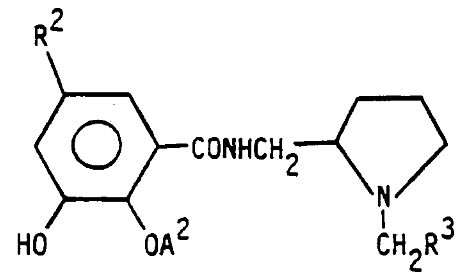

(dans laquelle $R^2$, $R^3$ et $A^2$ ont les définitions données ci-dessus) avec un composé de formule:

$$R^{1'''}—B$$

(dans laquelle $R^{1'''}$ a la définition donnée ci-dessus, et B représente un groupe partant), pour former un composé de formule $I^{ef}$, puis, si on le désire, on convertit le composé, obtenu par l'une des méthodes a)—b), en un de ses sels physiologiquement acceptables et/ou on le convertit en un de ses stéréoisomères essentiellement purs.

13. Procédé selon l'une quelconque des revendications 9 à 12, caractérisé en ce qu'on prépare un composé selon l'une quelconque des revendications 1 à 8.

14. Préparation pharmaceutique comprenant, à titre d'ingrédient actif, un composé selon l'une quelconque des revendications 1 à 8 ou un sel physiologiquement acceptable ou un isomère optique de ce composé.

15. Préparation pharmaceutique selon la revendication 14, sous forme d'unité dosée.

16. Préparation pharmaceutique selon les revendications 15—15, comprenant l'ingrédient actif en association avec un support, excipient ou véhicule pharmaceutiquement acceptable.

17. Utilisation d'un composé selon l'une quelconque des revendications 1 à 8 ou d'un de ses sels physiologiquement acceptables, pour la préparation d'une préparation pharmaceutique comprenant, à titre d'ingrédient actif, une certaine quantité dudit composé.

18. Composé selon l'une quelconque des revendications 1 à 8, pour l'utilisation comme médicament.

**Revendications pour l'Etat contractant: AT**

1. Procédé pour préparer un composé de formule:

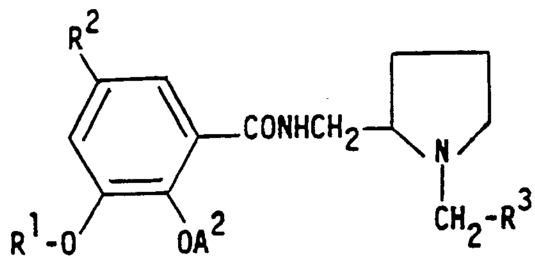

[dans laquelle:

$R^1$ représente un atome d'hydrogène, un groupe, un groupe alkyle ayant 1 à 4 atomes de C), un groupe alkyl (1 à 20C)-CO, un groupe alkyl (1 à 20 C)—O—CO, un groupe alkyl (1 à 20C)—NH—CO ou un groupe

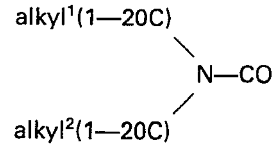

où les groupes alkyle[1] et alkyle[2] peuvent être identiques ou différents,

$R^2$ représente un atome d'halogène, un groupe alkyle (1 à 4C) ou un groupe alkyl (1 à 4C)-thio,

$A^2$ représente un groupe méthyle ou éthyle, et

$R^3$ représente un atome d'hydrogène, un groupe alkyle (1 à 4C), un groupe alcényle (2 ou 3C) ou un groupe phényle, substitué par un ou plusieurs groupes fluoro, chloro, bromo, trifluorométhyle, éthyle, méthoxy ou éthoxy en position(s) ortho, méta ou para, ou substitué par un groupe méthylènedioxy];

ou un sel physiologiquement acceptable ou un isomère optique de ce composé, ce procédé comprenant:

a) la réaction d'un composé de formule:

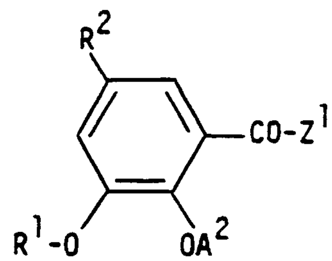

(dans laquelle $R^1$, $R^2$ et $A^2$ ont les définitions données ci-dessus, et —CO—$Z^1$ est un groupe réactif, capable de réagir avec un groupement amino en formant un fragment amide) avec un composé de formule:

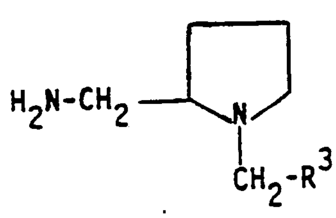

(dans laquelle $R^3$ a la définition donnée ci-dessus), ou avec un dérivé réactif de ce composé, pour former un composé de formule I, ou

b) la réaction de substitution sur l'azote d'un composé de formule:

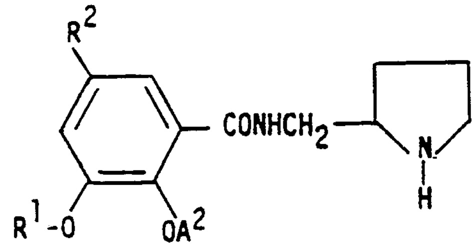

(dans laquelle $R^1$, $R^2$ et $A^2$ ont les définitions données ci-dessus) avec un composé de formule:

1) $R^3—CH_2—X$; ou

2) $R^3CHO$, en présence d'un agent réducteur; ou

3) $R^3COCl$, $(R^3CO)_2O$ ou $R^3—COOCH_3$, et réduction subséquente,

(où $R^3$ a la définition donnée ci-dessus, et X est un groupe partant) pour former un composé de formule I puis, si on le désire, on convertit le composé ainsi obtenu par l'une des méthodes a)—b) en un de sel physiologiquement acceptable de ce composé et/ou on le convertit en un de ses stéréoisomères essentiellement purs.

2. Procédé de préparation d'un composé de formule:

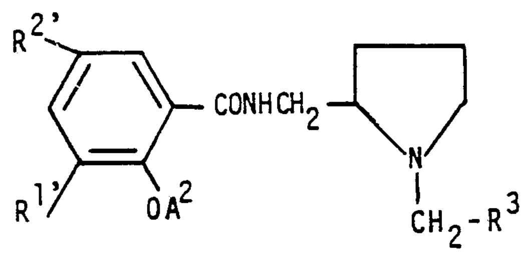

$I^c$

(dans laquelle $A^2$ et $R^2$ ont les définitions données à la revendication 1, et $R^{1'}$ représente un atome d'hydrogène ou un groupe alkyle (1 à 4C) et $R^{2'}$ représente un atome de brome ou de chlore) ou d'un sel physiologiquement acceptable ou d'un isomère optique de ce composé, ce procédé comprenant la réaction d'un composé de formule:

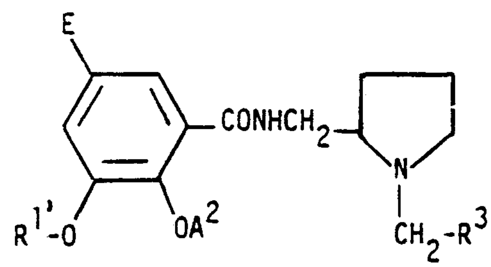

(dans laquelle $R^{1'}$, $A^2$ et $R^3$ ont les définitions données ci-dessus, et E représente un atome d'hydrogène ou un groupe $Si(CH_3)_3$), avec un agent de chloration ou de bromation, pour former un composé de formule $I^c$ puis, si on le désire, on convertit le composé ainsi obtenu en un de ses sels physiologiquement acceptables et/ou on le convertit en un de ses stéréoisomères essentiellement purs.

3. Procédé pour préparer un composé de formule:

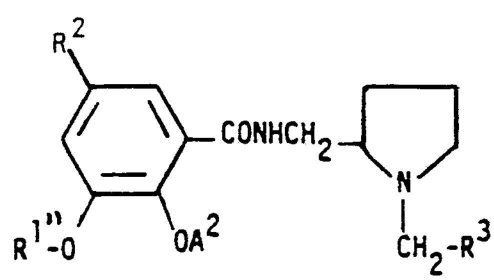

$I^d$

(dans laquelle $R^2$, $R^3$ et $A^2$ ont les définitions données à la revendication 1, et $R^{1''}$ représente un groupe alkyle (1 à 4C)), ou d'un de ses sels physiologiquemente acceptables ou un isomère optique de ce composé, ce procédé comprenant la réaction d'un composé de formule:

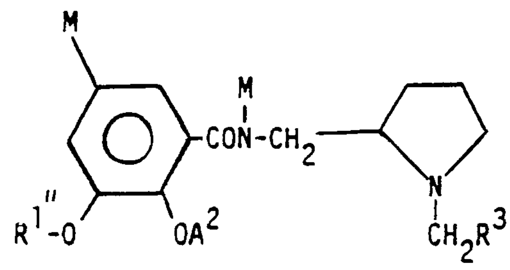

(dans laquelle $R^{1''}$, $R^3$ et $A^2$ ont les définitions données ci-dessus et M représente un dérivé de métal) avec un réactif électrophile, pour former un composé de formule $I^d$ puis, si on le désire, on convertit le composé ainsi obtenu en un de ses sels physiologiquement acceptables et/ou on le convertit en un de ses stéréoisomères essentiellement purs.

4. Procédé pour préparer un composé de formule:

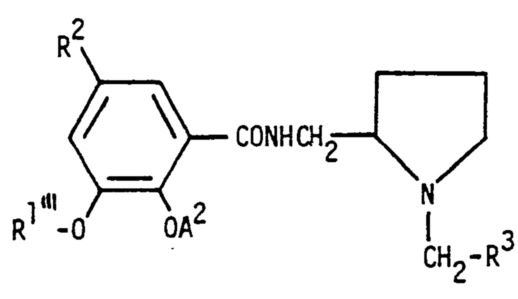

(dans laquelle $R^2$, $R^3$ et $A^2$ ont les définitions données à la revendication 1, et

$R^{1'''}$ représente un groupe alkyle (1 à 4C), un groupe alkyl (1 à 20C)-CO, un groupe alkyl (1 à 20 C)—O—CO, un groupe alkyl (1 à 20C)—NH—CO ou un groupe:

alkyl¹(1—20C)
\
N—CO,
/
alkyl²(1—20C)

ou un de ses sels physiologiquement acceptables ou un isomere optique de ce composé, ce procédé comprenant:

a) une O-alkylation d'un composé de formule:

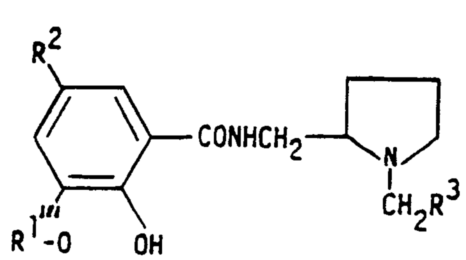

$I^{ef}$

(dans laquelle $R^{1'''}$, $R^2$ et $R^3$ ont les définitions données ci-dessus) avec un composé de formule:

$A^2$—B

(dans laquelle $A^2$ a la définition donnée ci-dessus et B est un groupe partant), pour former un composé de formule $I^{ef}$, ou

b) la réaction d'un composé de formule:

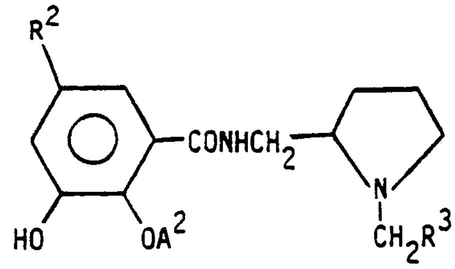

(dans laquelle $R^2$, $R^3$ et $A^2$ ont les définitions données ci-dessus) avec un composé de formule:

$$R^{1'''}—B$$

(dans laquelle $R^{1'''}$ a la définition donnée ci-dessus, et B est un groupe partant), pour former un composé de formule $I^{ef}$, puis, si on le désire, on convertit le composé, obtenu selon l'une des méthodes a)—b), en un de ses sels physiologiquement acceptables et/ou on le convertit en un de ses stéréoisomères essentiellement purs.

5. Procédé selon l'une quelconque des revendications 1 à 4, dans lequel on prépare un composé de formule I, dans laquelle:

$R^1$ représente un atome d'hydrogène, un groupe méthyle, éthyle ou alkyl (1 à 20C)-CO;
$R^2$ représente un atome d'halogène ou un groupe alkyle;
$A^2$ représente un groupe méthyle ou éthyle; et
$R^3$ a la définition indiquée à la revendication 1.

6. Procédé selon l'une quelconque des revendications 1 à 4, dans lequel on prépare un composé de formule I, dans laquelle:

$R^1$ représente un atome d'hydrogène, un groupe méthyle ou éthyle;
$R^2$ représente un atome de chlore, de brome, d'iode, un groupe méthyle, éthyle, n-propyle ou n-butyle;
$A^2$ représente un groupe méthyle ou éthyle; et
$R^3$ a la définition indiquée à la revendication 1.

7. Procédé selon l'une quelconque des revendications 1 à 4, dans lequel on prépare un composé de formule I, dans laquelle:

$R^1$ représente un groupe méthyle;
$R^2$ représente un atome de chlore, de brome, d'iode, un groupe méthyle, éthyle, n-propyle ou n-butyle;
$A^3$ représente un groupe méthyle; et
$R^3$ représente un atome d'hydrogène, un groupe alkyle (1 à 4C), un groupe alcényle (2—3C), ou un groupe phényle substitué en position para par un atome de fluor.

8. Procédé selon l'une quelconque des revendications 1 à 4, dans lequel on prépare un composé de formule I, dans laquelle:

$R^1$ représente un groupe méthyle;
$R^2$ représente un atome de brome;
$A^3$ représente un groupe méthyle; et
$R^3$ a la définition donnée à la revendication 7.

9. Procédé selon l'une quelconque des revendications 1 à 4, dans lequel on prépare un composé de formule:

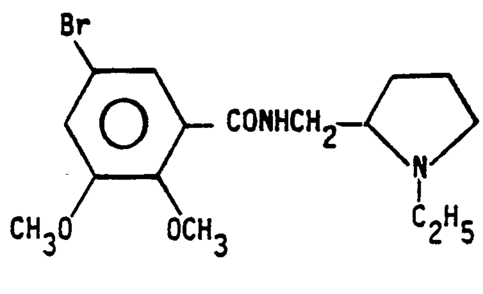

ou

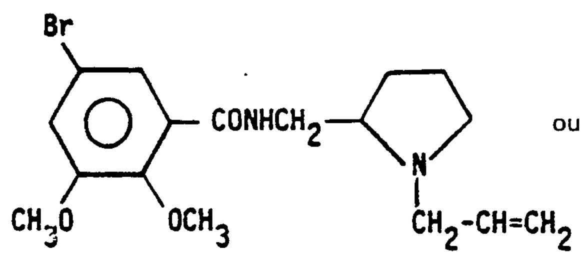

ou

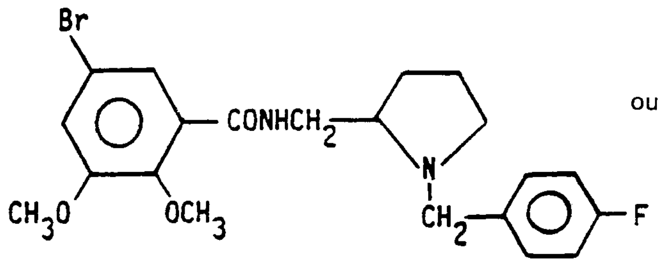

ou

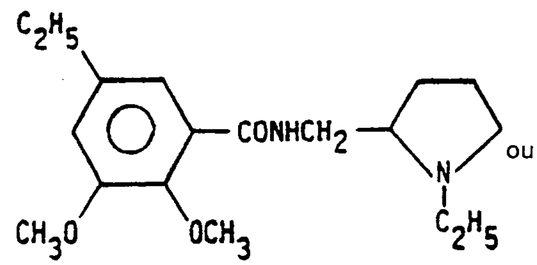

ou

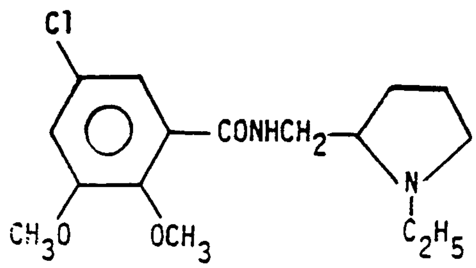

ou

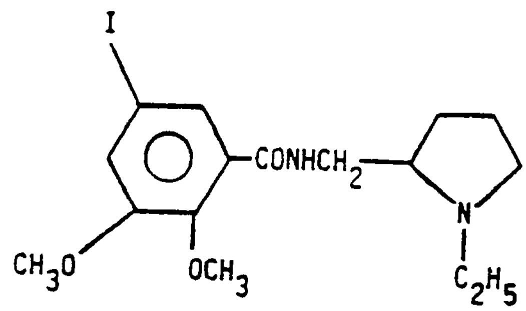

ou

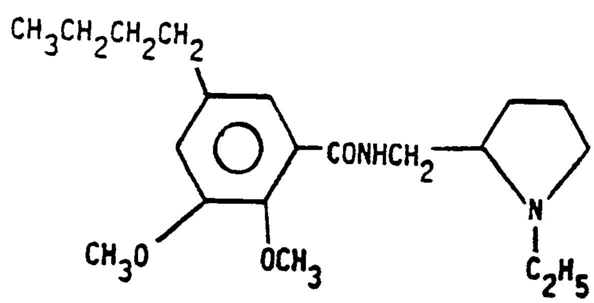

10. Procédé selon l'une quelconque des revendications 1 à 9, dans lequel on prépare le composé sous forme d'un isomère optique.

11. Procédé selon l'une quelconque des revendications 1 à 10, dans lequel on prépare le composé sous forme d'un sel physiologiquement acceptable.